# EUROPEAN PATENT APPLICATION

(11) **EP 1 407 774 A1**
(43) Date of publication of application: **14.04.2004**
(21) Application number: 02020255.2
(22) Date of filing: 10.09.2002
(51) Int. Cl.: A61K 31/517, C07D 239/95, C07D 401/12, C07D 403/04, A61P 3/06

(54) **2-Amino-4-quinazolinones as LXR nuclear receptor binding compounds**

(71) Applicant: LION bioscience AG, 69123 Heidelberg (DE)
(72) Inventor: Deuschle, Ulrich, 69245 Bammental (DE); Loebbert, Ralph, 69115 Heidelberg (DE); Blume, Beatrix, 69221 Dossenheim (DE); Koegl, Manfred, 69214 Eppenheim (DE); Kremoser, Claus, 69121 Heidelberg (DE); Kober, Ingo, 69251 Gaiberg (DE); Bauer, Ulrike, 69207 Sandhausen (DE); Giegrich, Kristina, 68623 Lampertheim (DE)
(74) Representative: Goddar, Heinz J., Dr.

(57) **Abstract**

The present invention relates to 2-amine-4-oxo-quinazolines which bind to the LXR receptors and act as agonists and antagonists of the LXR receptors. The invention further relates to the treatment of diseases and/or conditions through binding of said nuclear receptor by said compounds and the production of medicaments using said compounds. In particular the compounds are useful in the treatment of hypercholesterolemia, obesity or other diseases associated with elevated lipoprotein (LDL) levels.

SEQUENCE LISTING

<110> LION bioscience
<120> LXR Nuclear receptor binding compounds
<130> FB11712
<160> 8
<170> PatentIn version 3.1

<210> 1
<211> 52
<212> DNA
<213> Homo sapiens

<400> 1
ggggaccact ttgtacaaga aagctgggtc cccttctcag tctgttccac tt 52

<210> 2
<211> 1344
<212> DNA
<213> Homo sapiens

<400> 2
   atgtccttgt ggctgggggc ccctgtgcct gacattcctc ctgactctgc ggtggagctg 60
   tggaagccag gcgcacagga tgcaagcagc caggcccagg gaggcagcag ctgcatcctc 120
   agagaggaag ccaggatgcc ccactctgct gggggtactg caggggtggg gctggaggct 180
   gcagagccca cagccctgct caccagggca gagccccctt cagaacccac agagatccgt 240
   ccacaaaagc ggaaaaaggg gccagccccc aaaatgctgg ggaacgagct atgcagcgtg 300
   tgtggggaca aggcctcggg cttccactac aatgttctga gctgcgaggg ctgcaaggga 360
   ttcttccgcc gcagcgtcat caagggagcg cactacatct gccacagtgg cggccactgc 420
   cccatggaca cctacatgcg tcgcaagtgc caggagtgtc ggcttcgcaa atgccgtcag 480
   gctggcatgc gggaggagtg tgtcctgtca gaagaacaga tccgcctgaa gaaactgaag 540
   cggcaagagg aggaacaggc tcatgccaca tccttgcccc ccaggcgttc ctcacccccc 600
   caaatcctgc cccagctcag cccggaacaa ctgggcatga tcgagaagct cgtcgctgcc 660
   cagcaacagt gtaaccggcg ctccttttct gaccggcttc gagtcacgcc ttggcccatg 720
   gcaccagatc cccatagccg ggaggcccgt cagcagcgct ttgcccactt cactgagctg 780
   gccatcgtct ctgtgcagga gatagttgac tttgctaaac agctacccgg cttcctgcag 840
   ctcagccggg aggaccagat tgccctgctg aagacctctg cgatcgaggt gatgcttctg 900
   gagacatctc ggaggtacaa ccctgggagt gagagtatca ccttcctcaa ggatttcagt 960
   tataaccggg aagactttgc caaagcaggg ctgcaagtgg aattcatcaa ccccatcttc 1020
   gagttctcca gggccatgaa tgagctgcaa ctcaatgatg ccgagtttgc cttgctcatt 1080
   gctatcagca tcttctctgc agaccggccc aacgtgcagg accagctcca ggtggagagg 1140
   ctgcagcaca catatgtgga agccctgcat gcctacgtct ccatccacca tccccatgac 1200
   cgactgatgt tcccacggat gctaatgaaa ctggtgagcc tccggaccct gagcagcgtc 1260
   cactcagagc aagtgtttgc actgcgtctg caggacaaaa agctcccacc gctgctctct 1320
   gagatctggg atgtgcacga atga 1344

<210> 3
<211> 1263
<212> PRT
<213> Homo sapiens
<400> 3

Met Leu Val Lys Pro Leu Pro Asp Ser Glu Glu Glu Gly His Asp Asn
1               5                   10                  15

Gln Glu Ala His Gln Lys Tyr Glu Thr Met Gln Cys Phe Ala Val Ser
            20                  25                  30

Gln Pro Lys Ser Ile Lys Glu Glu Gly Glu Asp Leu Gln Ser Cys Leu
        35                  40                  45

Ile Cys Val Ala Arg Arg Val Pro Met Lys Glu Arg Pro Val Leu Pro
    50                  55                  60

Ser Ser Glu Ser Phe Thr Thr Arg Gln Asp Leu Gln Gly Lys Ile Thr
65                  70                  75                  80

Ser Leu Asp Thr Ser Thr Met Arg Ala Ala Met Lys Pro Gly Trp Glu
                85                  90                  95

Asp Leu Val Arg Arg Cys Ile Gln Lys Phe His Ala Gln His Glu Gly
            100                 105                 110

Glu Ser Val Ser Tyr Ala Lys Arg His His His Glu Val Leu Arg Gln
        115                 120                 125

Gly Leu Ala Phe Ser Gln Ile Tyr Arg Phe Ser Leu Ser Asp Gly Thr
    130                 135                 140

Leu Val Ala Ala Gln Thr Lys Ser Lys Leu Ile Arg Ser Gln Thr Thr
145                 150                 155                 160

Asn Glu Pro Gln Leu Val Ile Ser Leu His Met Leu His Arg Glu Gln
                165                 170                 175

Asn Val Cys Val Met Asn Pro Asp Leu Thr Gly Gln Thr Met Gly Lys
            180                 185                 190

Pro Leu Asn Pro Ile Ser Ser Asn Ser Pro Ala His Gln Ala Leu Cys
        195                 200                 205

Ser Gly Asn Pro Gly Gln Asp Met Thr Leu Ser Ser Asn Ile Asn Phe
    210                 215                 220

Pro Ile Asn Gly Pro Lys Glu Gln Met Gly Met Pro Met Gly Arg Phe
225                 230                 235                 240

Gly Gly Ser Gly Gly Met Asn His Val Ser Gly Met Gln Ala Thr Thr
                245                 250                 255

Pro Gln Gly Ser Asn Tyr Ala Leu Lys Met Asn Ser Pro Ser Gln Ser
            260                 265                 270

Ser Pro Gly Met Asn Pro Gly Gln Pro Thr Ser Met Leu Ser Pro Arg
        275                 280                 285

His Arg Met Ser Pro Gly Val Ala Gly Ser Pro Arg Ile Pro Pro Ser
    290                 295                 300

Gln Phe Ser Pro Ala Gly Ser Leu His Ser Pro Val Gly Val Cys Ser
305                 310                 315                 320

Ser Thr Gly Asn Ser His Ser Tyr Thr Asn Ser Ser Leu Asn Ala Leu
                325                 330                 335

Gln Ala Leu Ser Glu Gly His Gly Val Ser Leu Gly Ser Ser Leu Ala
            340                 345                 350

Ser Pro Asp Leu Lys Met Gly Asn Leu Gln Asn Ser Pro Val Asn Met
        355                 360                 365

Asn Pro Pro Pro Leu Ser Lys Met Gly Ser Leu Asp Ser Lys Asp Cys
    370                 375                 380

Phe Gly Leu Tyr Gly Glu Pro Ser Glu Gly Thr Thr Gly Gln Ala Glu
385                 390                 395                 400

Ser Ser Cys His Pro Gly Glu Gln Lys Glu Thr Asn Asp Pro Asn Leu
                405                 410                 415

Pro Pro Ala Val Ser Ser Glu Arg Ala Asp Gly Gln Ser Arg Leu His
            420                 425                 430

Asp Ser Lys Gly Gln Thr Lys Leu Leu Gln Leu Leu Thr Thr Lys Ser
        435                 440                 445

Asp Gln Met Glu Pro Ser Pro Leu Ala Ser Ser Leu Ser Asp Thr Asn

450                 455                 460

Lys Asp Ser Thr Gly Ser Leu Pro Gly Ser Gly Ser Thr His Gly Thr
465                 470                 475                 480

Ser Leu Lys Glu Lys His Lys Ile Leu His Arg Leu Leu Gln Asp Ser
                485                 490                 495

Ser Ser Pro Val Asp Leu Ala Lys Leu Thr Ala Glu Ala Thr Gly Lys
            500                 505                 510

Asp Leu Ser Gln Glu Ser Ser Ser Thr Ala Pro Gly Ser Glu Val Thr
        515                 520                 525

Ile Lys Gln Glu Pro Val Ser Pro Lys Lys Lys Glu Asn Ala Leu Leu
    530                 535                 540

Arg Tyr Leu Leu Asp Lys Asp Asp Thr Lys Asp Ile Gly Leu Pro Glu
545                 550                 555                 560

Ile Thr Pro Lys Leu Glu Arg Leu Asp Ser Lys Thr Asp Pro Ala Ser
                565                 570                 575

Asn Thr Lys Leu Ile Ala Met Lys Thr Glu Lys Glu Glu Met Ser Phe
            580                 585                 590

Glu Pro Gly Asp Gln Pro Gly Ser Glu Leu Asp Asn Leu Glu Glu Ile
        595                 600                 605

Leu Asp Asp Leu Gln Asn Ser Gln Leu Pro Gln Leu Phe Pro Asp Thr
    610                 615                 620

Arg Pro Gly Ala Pro Ala Gly Ser Val Asp Lys Gln Ala Ile Ile Asn
625                 630                 635                 640

Asp Leu Met Gln Leu Thr Ala Glu Asn Ser Pro Val Thr Pro Val Gly
                645                 650                 655

Ala Gln Lys Thr Ala Leu Arg Ile Ser Gln Ser Thr Phe Asn Asn Pro
            660                 665                 670

Arg Pro Gly Gln Leu Gly Arg Leu Leu Pro Asn Gln Asn Leu Pro Leu
        675                 680                 685

Asp Ile Thr Leu Gln Ser Pro Thr Gly Ala Gly Pro Phe Pro Pro Ile
    690                 695                 700

Arg Asn Ser Ser Pro Tyr Ser Val Ile Pro Gln Pro Gly Met Met Gly
705                 710                 715                 720

Asn Gln Gly Met Ile Gly Asn Gln Gly Asn Leu Gly Asn Ser Ser Thr
                725                 730                 735

Gly Met Ile Gly Asn Ser Ala Ser Arg Pro Thr Met Pro Ser Gly Glu
            740                 745                 750

Trp Ala Pro Gln Ser Ser Ala Val Arg Val Thr Cys Ala Ala Thr Thr
        755                 760                 765

Ser Ala Met Asn Arg Pro Val Gln Gly Gly Met Ile Arg Asn Pro Ala
    770                 775                 780

Ala Ser Ile Pro Met Arg Pro Ser Ser Gln Pro Gly Gln Arg Gln Thr
785                 790                 795                 800

Leu Gln Ser Gln Val Met Asn Ile Gly Pro Ser Glu Leu Glu Met Asn
                805                 810                 815

Met Gly Gly Pro Gln Tyr Ser Gln Gln Gln Ala Pro Pro Asn Gln Thr
            820                 825                 830

Ala Pro Trp Pro Glu Ser Ile Leu Pro Ile Asp Gln Ala Ser Phe Ala
        835                 840                 845

Ser Gln Asn Arg Gln Pro Phe Gly Ser Ser Pro Asp Asp Leu Leu Cys
    850                 855                 860

Pro His Pro Ala Ala Glu Ser Pro Ser Asp Glu Gly Ala Leu Leu Asp
865                 870                 875                 880

Gln Leu Tyr Leu Ala Leu Arg Asn Phe Asp Gly Leu Glu Glu Ile Asp
                885                 890                 895

Arg Ala Leu Gly Ile Pro Glu Leu Val Ser Gln Ser Gln Ala Val Asp
            900                 905                 910

Pro Glu Gln Phe Ser Ser Gln Asp Ser Asn Ile Met Leu Glu Gln Lys
        915                 920                 925

Ala Pro Val Phe Pro Gln Gln Tyr Ala Ser Gln Ala Gln Met Ala Gln
    930                 935                 940

Gly Ser Tyr Ser Pro Met Gln Asp Pro Asn Phe His Thr Met Gly Gln
945                 950                 955                 960

Arg Pro Ser Tyr Ala Thr Leu Arg Met Gln Pro Arg Pro Gly Leu Arg
                965                 970                 975

Pro Thr Gly Leu Val Gln Asn Gln Pro Asn Gln Leu Arg Leu Gln Leu
            980                 985                 990

Gln His Arg Leu Gln Ala Gln Gln Asn Arg Gln Pro Leu Met Asn Gln
        995                 1000                1005

Ile Ser Asn Val Ser Asn Val Asn Leu Thr Leu Arg Pro Gly Val
    1010                1015                1020

Pro Thr Gln Ala Pro Ile Asn Ala Gln Met Leu Ala Gln Arg Gln
    1025                1030                1035

Arg Glu Ile Leu Asn Gln His Leu Arg Gln Arg Gln Met His Gln
    1040                1045                1050

Gln Gln Gln Val Gln Gln Arg Thr Leu Met Met Arg Gly Gln Gly
    1055                1060                1065

Leu Asn Met Thr Pro Ser Met Val Ala Pro Ser Gly Met Pro Ala
    1070                1075                1080

Thr Met Ser Asn Pro Arg Ile Pro Gln Ala Asn Ala Gln Gln Phe
    1085                1090                1095

Pro Phe Pro Pro Asn Tyr Gly Ile Ser Gln Gln Pro Asp Pro Gly
    1100                1105                1110

Phe Thr Gly Ala Thr Thr Pro Gln Ser Pro Leu Met Ser Pro Arg
    1115                1120                1125

Met Ala His Thr Gln Ser Pro Met Met Gln Gln Ser Gln Ala Asn
    1130                1135                1140

Pro Ala Tyr Gln Ala Pro Ser Asp Ile Asn Gly Trp Ala Gln Gly
    1145                1150                1155

Asn Met Gly Gly Asn Ser Met Phe Ser Gln Gln Ser Pro Pro His
    1160                1165                1170

Phe Gly Gln Gln Ala Asn Thr Ser Met Tyr Ser Asn Asn Met Asn
    1175                1180                1185

Ile Asn Val Ser Met Ala Thr Asn Thr Gly Gly Met Ser Ser Met
    1190                1195                1200

Asn Gln Met Thr Gly Gln Ile Ser Met Thr Ser Val Thr Ser Val
    1205                1210                1215

Pro Thr Ser Gly Leu Ser Ser Met Gly Pro Glu Gln Val Asn Asp
    1220                1225                1230

Pro Ala Leu Arg Gly Gly Asn Leu Phe Pro Asn Gln Leu Pro Gly
    1235                1240                1245

Met Asp Met Ile Lys Gln Glu Gly Asp Thr Thr Arg Lys Tyr Cys
    1250                1255                1260

<210> 4
<211> 6158
<212> DNA
<213> Homo sapiens

<400> 4
ggcggccgca gcctcggcta cagcttcggc ggcgaaggtc agcgccgacg gcagccggca      60

cctgacggcg tgaccgaccc gagccgattt ctcttggatt tggctacaca cttatagatc     120

ttctgcactg tttacaggca cagttgctga tatgtgttca agatgagtgg gatgggagaa     180

aatacctctg acccctccag ggcagagaca agaaagcgca aggaatgtcc tgaccaactt     240

ggacccagcc ccaaaaggaa cactgaaaaa cgtaatcgtg aacaggaaaa taaatatata     300

gaagaacttg cagagttgat ttttgcaaat tttaatgata tagacaactt taacttcaaa     360

cctgacaaat gtgcaatctt aaaagaaact gtgaagcaaa ttcgtcagat caaagaacaa     420

gagaaagcag cagctgccaa catagatgaa gtgcagaagt cagatgtatc ctctacaggg     480

cagggtgtca tcgacaagga tgcgctgggg cctatgatgc ttgaggccct tgatgggttc     540

ttctttgtag tgaacctgga aggcaacgtt gtgtttgtgt cagagaatgt gacacagtat     600

ctaaggtata accaagaaga gctgatgaac aaaagtgtat atagcatctt gcatgttggg     660

gaccacacgg aatttgtcaa aaacctgctg ccaaagtcta taggtaaatg ggggatcttg     720

gtctggcgaa cctccgaggc ggaacagcca taccttcaat tgtcggatgc tggtaaaacc     780

tttacctgat tcagaagagg agggtcatga taaccaggaa gctcatcaga aatatgaaac     840

tatgcagtgc ttcgctgtct ctcaaccaaa gtccatcaaa gaagaaggag aagatttgca     900

gtcctgcttg atttgcgtgg caagaagagt tcccatgaag gaaagaccag ttcttccctc     960

atcagaaagt tttactactc gccaggatct ccaaggcaag atcacgtctc tggataccag    1020

caccatgaga gcagccatga aaccaggctg ggaggacctg gtaagaaggt gtattcagaa    1080

gttccatgcg cagcatgaag gagaatctgt gtcctatgct aagaggcatc atcatgaagt    1140

actgagacaa ggattggcat tcagtcaaat ctatcgtttt tccttgtctg atggcactct    1200

tgttgctgca caaacgaaga gcaaactcat ccgttctcag actactaatg aacctcaact 1260
tgtaatatct ttacatatgc ttcacagaga gcagaatgtg tgtgtgatga atccggatct 1320
gactggacaa acgatgggga agccactgaa tccaattagc tctaacagcc ctgcccatca 1380
ggccctgtgc agtgggaacc caggtcagga catgaccctc agtagcaata taaattttcc 1440
cataaatggc ccaaaggaac aaatgggcat gcccatgggc aggtttggtg gttctggggg 1500
aatgaaccat gtgtcaggca tgcaagcaac cactcctcag ggtagtaact atgcactcaa 1560
aatgaacagc ccctcacaaa gcagccctgg catgaatcca ggacagccca cctccatgct 1620
ttcaccaagg catcgcatga gccctggagt ggctggcagc cctcgaatcc cacccagtca 1680
gttttcccct gcaggaagct tgcattcccc tgtgggagtt tgcagcagca caggaaatag 1740
ccatagttat accaacagct ccctcaatgc acttcaggcc ctcagcgagg ggcacggggt 1800
ctcattaggg tcatcgttgg cttcaccaga cctaaaaatg ggcaatttgc aaaactcccc 1860
agttaatatg aatcctcccc cactcagcaa gatgggaagc ttggactcaa aagactgttt 1920
tggactatat ggggagccct ctgaaggtac aactggacaa gcagagagca gctgccatcc 1980
tggagagcaa aaggaaacaa atgaccccaa cctgcccccg gccgtgagca gtgagagagc 2040
tgacgggcag agcagactgc atgacagcaa agggcagacc aaactcctgc agctgctgac 2100
caccaaatct gatcagatgg agccctcgcc cttagccagc tctttgtcgg atacaaacaa 2160
agactccaca ggtagcttgc ctggttctgg gtctacacat ggaacctcgc tcaaggagaa 2220
gcataaaatt ttgcacagac tcttgcagga cagcagttcc cctgtggact tggccaagtt 2280
aacagcagaa gccacaggca aagacctgag ccaggagtcc agcagcacag ctcctggatc 2340
agaagtgact attaaacaag agccggtgag ccccaagaag aaagagaatg cactacttcg 2400
ctatttgcta gataaagatg atactaaaga tattggttta ccagaaataa cccccaaact 2460
tgagagactg gacagtaaga cagatcctgc cagtaacaca aaattaatag caatgaaaac 2520
tgagaaggag gagatgagct ttgagcctgg tgaccagcct ggcagtgagc tggacaactt 2580
ggaggagatt ttggatgatt tgcagaatag tcaattacca cagcttttcc cagacacgag 2640
gccaggcgcc cctgctggat cagttgacaa gcaagccatc atcaatgacc tcatgcaact 2700
cacagctgaa aacagccctg tcacacctgt tggagcccag aaaacagcac tgcgaatttc 2760
acagagcact tttaataacc cacgaccagg gcaactgggc aggttattgc caaaccagaa 2820
tttaccactt gacatcacat tgcaaagccc aactggtgct ggacctttcc caccaatcag 2880
aaacagtagt ccctactcag tgatacctca gccaggaatg atgggtaatc aagggatgat 2940
aggaaaccaa ggaaatttag ggaacagtag cacaggaatg attggtaaca gtgcttctcg 3000
gcctactatg ccatctggag aatgggcacc gcagagttcg gctgtgagag tcacctgtgc 3060

tgctaccacc agtgccatga accggccagt ccaaggaggt atgattcgga acccagcagc 3120

cagcatcccc atgaggccca gcagccagcc tggccaaaga cagacgcttc agtctcaggt 3180

catgaatata gggccatctg aattagagat gaacatgggg ggacctcagt atagccaaca 3240

acaagctcct ccaaatcaga ctgccccatg gcctgaaagc atcctgccta tagaccaggc 3300

gtcttttgcc agccaaaaca ggcagccatt tggcagttct ccagatgact tgctatgtcc 3360

acatcctgca gctgagtctc cgagtgatga gggagctctc ctggaccagc tgtatctggc 3420

cttgcggaat tttgatggcc tggaggagat tgatagagcc ttaggaatac ccgaactggt 3480

cagccagagc caagcagtag atccagaaca gttctcaagt caggattcca acatcatgct 3540

ggagcagaag gcgcccgttt tcccacagca gtatgcatct caggcacaaa tggcccaggg 3600

tagctattct cccatgcaag atccaaactt tcacaccatg ggacagcggc ctagttatgc 3660

cacactccgt atgcagccca gaccgggcct caggcccacg ggcctagtgc agaaccagcc 3720

aaatcaacta agacttcaac ttcagcatcg cctccaagca cagcagaatc gccagccact 3780

tatgaatcaa atcagcaatg tttccaatgt gaacttgact ctgaggcctg gagtaccaac 3840

acaggcacct attaatgcac agatgctggc ccagagacag agggaaatcc tgaaccagca 3900

tcttcgacag agacaaatgc atcagcaaca gcaagttcag caacgaactt tgatgatgag 3960

aggacaaggg ttgaatatga caccaagcat ggtggctcct agtggtatgc cagcaactat 4020

gagcaaccct cggattcccc aggcaaatgc acagcagttt ccatttcctc caaactacgg 4080

aataagtcag caacctgatc caggctttac tggggctacg actccccaga gcccacttat 4140

gtcaccccga atggcacata cacagagtcc catgatgcaa cagtctcagg ccaacccagc 4200

ctatcaggcc ccctccgaca taaatggatg ggcgcagggg aacatggcgg gaaacagcat 4260

gttttcccag cagtccccac cacactttgg gcagcaagca aacaccagca tgtacagtaa 4320

caacatgaac atcaatgtgt ccatggcgac caacacaggt ggcatgagca gcatgaacca 4380

gatgacagga cagatcagca tgacctcagt gacctccgtg cctacgtcag ggctgtcctc 4440

catgggtccc gagcaggtta atgatcctgc tctgagggga ggcaacctgt tcccaaacca 4500

gctgcctgga atggatatga ttaagcagga gggagacaca acacggaaat attgctgaca 4560

ctgctgaagc cagttgcttc ttcagctgac cgggctcact tgctcaaaac acttccagtc 4620

tggagagctg tgtctatttg tttcaaccca actgacctgc cagccggttc tgctagagca 4680

gacaggcctg gccctggttc ccagggtggc gtccactcgg ctgtggcagg aggagctgcc 4740

tcttctcttg acagtctgaa gctcgcatcc agacagtcgc tcagtctgtt cactgcattc 4800

accttagtgc aacttagatc tctcctgcaa aagtaaatgt tgacaggcaa atttcatacc 4860

catgtcagat tgaatgtatt taaatgtatg tatttaagga gaaccatgct cttgttctgt 4920

tcctgttcgg ttccagacac tggtttcttg ctttgttttc cctggctaac agtctagtgc 4980

aaaagattaa gattttatct gggggaaaga aaagaatttt ttaaaaaatt aaactaaaga 5040

tgttttaagc taaagcctga atttgggatg gaagcaggac agacaccgtg gacagcgctg 5100

tatttacaga cacacccagt gcgtgaagac caacaaagtc acagtcgtat ctctagaaag 5160

ctctaaagac catgttggaa agagtctcca gttactgaac agatgaaaag gagcctgtga 5220

gagggctgtt aacattagca aatatttttt ccttgttttt tctttgttaa aaccaaactg 5280

gttcacctga atcatgaatt gagaagaaat aattttcatt tctaaattaa gtccctttta 5340

gtttgatcag acagcttgaa tcagcatctc ttcttccctg tcagcctgac tcttcccttc 5400

ccctctctca ttccccatac tccctatttt cattcctttt ttaaaaaata atataagcta 5460

cagaaaccag gtaagccctt tatttcctta aatgttttgc cagccactta ccaattgcta 5520

agtattgaat ttcagaaaaa aaaaatgcat ttactggcaa ggagaagagc aaagttaagg 5580

cttgatacca atcgagctaa ggatacctgc tttggaagca tgtttattct gttccccagc 5640

aactctggcc tccaaaatgg gagaaaacgc cagtgtgttt aaattgatag cagatatcac 5700

gacagattta acctctgcca tgtgtttttt attttgtttt ttagcagtgc tgactaagcc 5760

gaagttttgt aaggtacata aaatccaatt tatatgtaaa caagcaataa tttaagttga 5820

gaacttatgt gttttaattg tataattttt gtgaggtata catattgtgg aattgactca 5880

aaaatgaggt acttcagtat taaattagat atcttcatag caatgtctcc taaaggtgtt 5940

ttgtaaagga tatcaatgcc ttgattagac ctaatttgta gacttaagac tttttatttt 6000

ctaaaccttg tgattctgct tataagtcat ttatctaatc tatatgatat gcagccgctg 6060

taggaaccaa ttcttgattt ttatatgttt atattctttc ttaatgaacc ttagaaagac 6120

tacatgttac taagcaggcc acttttatgg ttgttttt 6158

<210> 5
<211> 460
<212> PRT
<213> Homo sapiens
<400> 5

Met Ser Ser Pro Thr Thr Ser Ser Leu Asp Thr Pro Leu Pro Gly Asn
1               5                   10                  15

Gly Pro Pro Gln Pro Gly Ala Pro Ser Ser Ser Pro Thr Val Lys Glu
            20                  25                  30

Glu Gly Pro Glu Pro Trp Pro Gly Gly Pro Asp Pro Asp Val Pro Gly
        35                  40                  45

Thr Asp Glu Ala Ser Ser Ala Cys Ser Thr Asp Trp Val Ile Pro Asp
    50                  55                  60

Pro Glu Glu Glu Pro Glu Arg Lys Arg Lys Lys Gly Pro Ala Pro Lys
65                  70                  75                  80

Met Leu Gly His Glu Leu Cys Arg Val Cys Gly Asp Lys Ala Ser Gly
                85                  90                  95

Phe His Tyr Asn Val Leu Ser Cys Glu Gly Cys Lys Gly Phe Phe Arg
            100                 105                 110

Arg Ser Val Val Arg Gly Gly Ala Arg Arg Tyr Ala Cys Arg Gly Gly
        115                 120                 125

Gly Thr Cys Gln Met Asp Ala Phe Met Arg Arg Lys Cys Gln Gln Cys
    130                 135                 140

Arg Leu Arg Lys Cys Lys Glu Ala Gly Met Arg Glu Gln Cys Val Leu
145                 150                 155                 160

Ser Glu Glu Gln Ile Arg Lys Lys Lys Ile Arg Lys Gln Gln Gln Glu
                165                 170                 175

Ser Gln Ser Gln Ser Gln Ser Pro Val Gly Pro Gln Gly Ser Ser Ser
            180                 185                 190

Ser Ala Ser Gly Pro Gly Ala Ser Pro Gly Gly Ser Glu Ala Gly Ser
        195                 200                 205

Gln Gly Ser Gly Glu Gly Glu Gly Val Gln Leu Thr Ala Ala Gln Glu
    210                 215                 220

Leu Met Ile Gln Gln Leu Val Ala Ala Gln Leu Gln Cys Asn Lys Arg
225                 230                 235                 240

Ser Phe Ser Asp Gln Pro Lys Val Thr Pro Trp Pro Leu Gly Ala Asp
                245                 250                 255

Pro Gln Ser Arg Asp Ala Arg Gln Gln Arg Phe Ala His Phe Thr Glu
            260                 265                 270

Leu Ala Ile Ile Ser Val Gln Glu Ile Val Asp Phe Ala Lys Gln Val
        275                 280                 285

Pro Gly Phe Leu Gln Leu Gly Arg Glu Asp Gln Ile Ala Leu Leu Lys
    290                 295                 300

Ala Ser Thr Ile Glu Ile Met Leu Leu Glu Thr Ala Arg Arg Tyr Asn
305                 310                 315                 320

His Glu Thr Glu Cys Ile Thr Phe Leu Lys Asp Phe Thr Tyr Ser Lys
                325                 330                 335

Asp Asp Phe His Arg Ala Gly Leu Gln Val Glu Phe Ile Asn Pro Ile
            340                 345                 350

Phe Glu Phe Ser Arg Ala Met Arg Arg Leu Gly Leu Asp Asp Ala Glu
        355                 360                 365

Tyr Ala Leu Leu Ile Ala Ile Asn Ile Phe Ser Ala Asp Arg Pro Asn
    370                 375                 380

Val Gln Glu Pro Gly Arg Val Glu Ala Leu Gln Gln Pro Tyr Val Glu
385                 390                 395                 400

Ala Leu Leu Ser Tyr Thr Arg Ile Lys Arg Pro Gln Asp Gln Leu Arg
                405                 410                 415

Phe Pro Arg Met Leu Met Lys Leu Val Ser Leu Arg Thr Leu Ser Ser
            420                 425                 430

Val His Ser Glu Gln Val Phe Ala Leu Arg Leu Gln Asp Lys Lys Leu
        435                 440                 445

Pro Pro Leu Leu Ser Glu Ile Trp Asp Val His Glu
    450                 455                 460

<210>  6
<211>  1383
<212>  DNA
<213>  Homo sapiens
<400>  6
atgtcctctc ctaccacgag ttccctggat acccccctgc ctggaaatgg cccccctcag      60

cctggcgccc cttcttcttc acccactgta aaggaggagg gtccggagcc gtggcccggg     120

ggtccggacc ctgatgtccc aggcactgat gaggccagct cagcctgcag cacagactgg     180

gtcatcccag atcccgaaga ggaaccagag cgcaagcgaa agaagggccc agccccgaag     240

atgctgggcc acgagctttg ccgtgtctgt ggggacaagg cctccggctt ccactacaac     300

gtgctcagct gcgaaggctg caagggcttc ttccggcgca gtgtggtccg tggtggggcc     360

aggcgctatg cctgccgggg tggcggaacc tgccagatgg acgctttcat gcggcgcaag     420

tgccagcagt gccggctgcg caagtgcaag gaggcaggga tgagggagca gtgcgtcctt     480

tctgaagaac agatccggaa gaagaagatt cggaaacagc agcaggagtc acagtcacag     540

tcgcagtcac ctgtggggcc gcagggcagc agcagctcag cctctgggcc tggggcttcc     600

cctggtggat ctgaggcagg cagccagggc tccggggaag gcgagggtgt ccagctaaca 660

gcggctcaag aactaatgat ccagcagttg gtggcggccc aactgcagtg caacaaacgc 720

tccttctccg accagcccaa agtcacgccc tggcccctgg gcgcagaccc ccagtcccga 780

gatgcccgcc agcaacgctt tgcccacttc acggagctgg ccatcatctc agtccaggag 840

atcgtggact tcgctaagca agtgcctggt ttcctgcagc tgggccggga ggaccagatc 900

gccctcctga aggcatccac tatcgagatc atgctgctag agacagccag gcgctacaac 960

cacgagacag agtgtatcac cttcttgaag gacttcacct acagcaagga cgacttccac 1020

cgtgcaggcc tgcaggtgga gttcatcaac cccatcttcg agttctcgcg ggccatgcgg 1080

cggctgggcc tggacgacgc tgagtacgcc ctgctcatcg ccatcaacat cttctcggcc 1140

gaccggccca acgtgcagga gccgggccgc gtggaggcgt tgcagcagcc ctacgtggag 1200

gcgctgctgt cctacacgcg catcaagagg ccgcaggacc agctgcgctt cccgcgcatg 1260

ctcatgaagc tggtgagcct gcgcacgctg agctctgtgc actcggagca ggtcttcgcc 1320

ttgcggctcc aggacaagaa gctgccgcct ctgctgtcgg agatctggga cgtccacgag 1380

tga 1383

<210> 7
<211> 49
<212> DNA
<213> Homo sapiens

<400> 7
ggggacaagt ttgtacaaaa aagcaggctc gcttcgcaaa tgccgtcag 49

<210> 8
<211> 447
<212> PRT
<213> Homo sapiens
<400> 8

Met Ser Leu Trp Leu Gly Ala Pro Val Pro Asp Ile Pro Pro Asp Ser
1               5                   10                  15

Ala Val Glu Leu Trp Lys Pro Gly Ala Gln Asp Ala Ser Ser Gln Ala
            20                  25                  30

Gln Gly Gly Ser Ser Cys Ile Leu Arg Glu Glu Ala Arg Met Pro His
        35                  40                  45

Ser Ala Gly Gly Thr Ala Gly Val Gly Leu Glu Ala Ala Glu Pro Thr
    50                  55                  60

Ala Leu Leu Thr Arg Ala Glu Pro Pro Ser Glu Pro Thr Glu Ile Arg
65                  70                  75                  80

Pro Gln Lys Arg Lys Lys Gly Pro Ala Pro Lys Met Leu Gly Asn Glu
                85                  90                  95

Leu Cys Ser Val Cys Gly Asp Lys Ala Ser Gly Phe His Tyr Asn Val
            100                 105                 110

Leu Ser Cys Glu Gly Cys Lys Gly Phe Phe Arg Arg Ser Val Ile Lys
        115                 120                 125

Gly Ala His Tyr Ile Cys His Ser Gly Gly His Cys Pro Met Asp Thr
    130                 135                 140

Tyr Met Arg Arg Lys Cys Gln Glu Cys Arg Leu Arg Lys Cys Arg Gln
145                 150                 155                 160

Ala Gly Met Arg Glu Glu Cys Val Leu Ser Glu Glu Gln Ile Arg Leu
                165                 170                 175

Lys Lys Leu Lys Arg Gln Glu Glu Glu Gln Ala His Ala Thr Ser Leu
            180                 185                 190

Pro Pro Arg Arg Ser Ser Pro Pro Gln Ile Leu Pro Gln Leu Ser Pro
        195                 200                 205

Glu Gln Leu Gly Met Ile Glu Lys Leu Val Ala Ala Gln Gln Gln Cys
    210                 215                 220

Asn Arg Arg Ser Phe Ser Asp Arg Leu Arg Val Thr Pro Trp Pro Met
225                 230                 235                 240

Ala Pro Asp Pro His Ser Arg Glu Ala Arg Gln Gln Arg Phe Ala His
                245                 250                 255

Phe Thr Glu Leu Ala Ile Val Ser Val Gln Glu Ile Val Asp Phe Ala
            260                 265                 270

Lys Gln Leu Pro Gly Phe Leu Gln Leu Ser Arg Glu Asp Gln Ile Ala
        275                 280                 285

Leu Leu Lys Thr Ser Ala Ile Glu Val Met Leu Leu Glu Thr Ser Arg
    290                 295                 300

Arg Tyr Asn Pro Gly Ser Glu Ser Ile Thr Phe Leu Lys Asp Phe Ser
305                 310                 315                 320

Tyr Asn Arg Glu Asp Phe Ala Lys Ala Gly Leu Gln Val Glu Phe Ile
                325                 330                 335

Asn Pro Ile Phe Glu Phe Ser Arg Ala Met Asn Glu Leu Gln Leu Asn
            340                 345                 350

Asp Ala Glu Phe Ala Leu Leu Ile Ala Ile Ser Ile Phe Ser Ala Asp
        355                 360                 365

Arg Pro Asn Val Gln Asp Gln Leu Gln Val Glu Arg Leu Gln His Thr
    370                 375                 380

Tyr Val Glu Ala Leu His Ala Tyr Val Ser Ile His His Pro His Asp
385                 390                 395                 400

Arg Leu Met Phe Pro Arg Met Leu Met Lys Leu Val Ser Leu Arg Thr
                405                 410                 415

Leu Ser Ser Val His Ser Glu Gln Val Phe Ala Leu Arg Leu Gln Asp
            420                 425                 430

Lys Lys Leu Pro Pro Leu Leu Ser Glu Ile Trp Asp Val His Glu
        435                 440                 445

## Description

The present invention relates to compounds according to the general formula (1), which bind to the LXR receptors and act as agonists and antagonists of the LXR receptors. The invention further relates to the treatment of diseases and/or conditions through binding of said nuclear receptor by said compounds and the production of medicaments using said compounds.

### BACKGROUND OF THE INVENTION

Multicellular organisms are dependent on advanced mechanisms of information transfer between cells and body compartments. The information that is transmitted can be highly complex and can result in the alteration of genetic programs involved in cellular differentiation, proliferation, or reproduction. The signals, or hormones, are often simple molecules, such as peptides, fatty acid, or cholesterol derivatives.

Many of these signals produce their effects by ultimately changing the transcription of specific genes. One well-studied group of proteins that mediate a cells response to a variety of signals is the family of transcription factors known as nuclear receptors, hereinafter referred to often as "NR". Members of this group include receptors for steroid hormones, vitamin D, ecdysone, cis and trans retinoic acid, thyroid hormone, bile acids, cholesterol-derivatives, fatty acids (and other peroxisomal proliferators), as well as so-called orphan receptors, proteins that are structurally similar to other members of this group, but for which no ligands are known (Escriva, H. et al., Ligand binding was acquired during evolution of nuclear receptors, PNAS, 94, 6803 - 6808, 1997). Orphan receptors may be indicative of unknown signaling pathways in the cell or may be nuclear receptors that function without ligand activation. The activation of transcription by some of these orphan receptors may occur in the absence of an exogenous ligand and/or through signal transduction pathways originating from the cell surface (Mangelsdorf, D. J. et al., The nuclear receptor superfamily: the second decade, Cell 83, 835-839, 1995).

In general, three functional domains have been defined in NRs. An amino terminal domain is believed to have some regulatory function. A DNA-binding domain hereinafter referred to as "DBD" usually comprises two zinc finger elements and recognizes a specific Hormone Responsive Element hereinafter referred to as "HRE" within the promoters of responsive genes. Specific amino acid residues in the "DBD" have been shown to confer DNA sequence binding specificity (Schena, M. & Yamamoto, K.R., Mammalian Glucocorticoid Receptor Derivatives Enhance Transcription in Yeast, Science, 241:965-967, 1988). A Ligand-binding-domain hereinafter referred to as "LBD" is at the carboxy-terminal region of known NRs. In the absence of hormone, the LBD of some but not all NRs appears to interfere with the interaction of the DBD with its HRE. Hormone binding seems to result in a conformational change in the NR and thus opens this interference (Brzozowski et al., Molecular basis of agonism and antagonism in the oestrogen receptor, Nature, 389, 753 - 758, 1997; Wagner et al., A structural role for hormone in the thyroid hormone receptor, Nature, 378, 690 - 697. 1995). A NR without the HBD constitutively activates transcription but at a low level.

Coactivators or transcriptional activators are proposed to bridge between sequence specific transcription factors and the basal transcription machinery and in addition to influence the chromatin structure of a target cell. Several proteins like SRC-1, ACTR, and Grip1 interact with NRs in a ligand enhanced manner (Heery et al., A signature motif in transcriptional coactivators mediates binding to nuclear receptors, Nature, 387, 733 - 736; Heinzel et al., A complex containing N-CoR, mSin3 and histone deacetylase mediates transcriptional repression, Nature 387, 43 - 47, 1997). Furthermore, the physical interaction with repressing receptor-interacting proteins or corepressors has been demonstrated (Xu et al., Coactivator and Corepressor complexes in nuclear receptor function, Curr Opin Genet Dev, 9 (2), 140 - 147, 1999).

Nuclear receptor modulators like steroid hormones affect the growth and function of specific cells by binding to intracellular receptors and forming nuclear receptor-ligand complexes. Nuclear receptor-hormone complexes then interact with a hormone response element (HRE) in the control region of specific genes and alter specific gene expression.

The term LXR (Liver X Receptor) includes all subtypes of this receptor. Specifically LXR includes LXRa (also known as LXRalpha, RLD-1 and NR1H3) and LXRb (also known as LXRbeta, NER, NER1, UR, OR-1, R1P15 and NH1H2) and ligands of LXR should be understood to include ligands of LXRa or LXRb. LXR is a prototypical type 2 nuclear receptor which activates genes upon binding to promoter region of target genes in a prototypical heterodimeric fashion with Retinoid X Receptor (hereinafter RXR, Forman et al., Cell, 81, 687-93, 1995). The relevant physiological ligands of LXR seem to be oxidized derivatives of cholesterol, including 22-hydroxycholesterol and 24,25(S)-epoxycholesterol (Lehmann, et al., Biol. Chem. 272(6), 3137-40, 1997). The oxysterol ligands bound to LXR were found to regulate the expression of several genes that participate in cholesterol metabolism (Janowski, et al., Nature, 383, 728-31, 1996).

LXR is proposed to be a hepatic oxysterol sensor. Upon activation (e.g. binding of oxysterols) it influences the conversion of dietary cholesterol into bile acids by upregulating the transcription of key genes which are involved in bile acid synthesis such as CYP7A1. Hence, activation of LXR in the liver could result in an increased synthesis of bile acids from cholesterol which could lead to decreased levels of hepatic cholesterol. This proposed LXR function in hepatic cholesterol metabolism was experimentally confirmed using knockout mice. Mice lacking the receptor LXRa lost their ability to respond normally to an increase in dietary cholesterol and did not induce transcription of the gene encoding CYP7A1. This resulted in accumulation of large quantities of cholesterol in the livers and impaired hepatic function. (Peet, et al., Cell, 93, 693-704, 1998).

Besides its important function in liver, LXR plays an important role in the regulation of cholesterol homeostasis in macrophages and intestinal mucosa cells where it upregulates cholesterol transporters from the ABC (=ATP binding cassette) family of membrane proteins (Repa, et al., J Biol Chem. 2002 May 24;277(21):18793-800). These transporters are believed to be crucially involved in the uptake of cholesterol from the diet since mutations in their genes leads to diseases such as sitosterolemia (Berge, et al., Science (2000);290(5497):1771-5.).

Other members of the ABC transporter family seem to be responsible for the efflux of cholesterol from loaded macrophages, a process which is thought to prevent the generation of atherosclerotic lesions. Stimulation of LXR by synthetic ligands might result in an increased cholesterol efflux from macrophages and a decreased deposition of atherosclerotic plaques (Venkateswaran, et al., PNAS (2000) 24;97(22):12097-102; Sparrow, et al., J Biol Chem (2002) 277(12):10021-7; Joseph, et al., PNAS (2002);99(11):7604-9).

However, in animal studies it was observed that activation of LXR in the liver by full agonists does not only increase bile acid synthesis but also stimulates the de novo synthesis of fatty acids and triglygerids through the upregulation of key enzymes such as Fatty Acid Synthase (FAS) or Stearyl-CoA Desaturase (SCD-1). (Schultz, et al., Genes Dev (2000) 14(22):2831-8.

Therefore, an ideal synthetic LXR binding compound should have properties that retain the agonistic activity on hepatic bile acid formation and ABC-transporter -mediated decrease in cholesterol uptake from the diet and increased cholesterol efflux from macrophages. In parallel such a compound should lack the hyperlipidemic potential which is exerted through increased fatty acid and triclyceride synthesis.

To date few compounds have been described which bind the LXR receptor and thus show utility for treating diseases or conditions which are due to or influenced by said nuclear receptor (Collins, et al., J Med Chem. (2002) 45(10):1963-6; Schultz, et al., Genes Dev (2000) 14(22):2831-8; Sparrow, et al., J Biol Chem (2002) 277(12):10021-7).

It was thus an object of the present invention to provide for compounds which by means of binding the LXR receptor act as agonist, antagonist or mixed agonist / antagonist of said receptor and thus show utility for treating diseases or conditions which are due to or influenced by said nuclear receptor.

It was further an object of the invention to provide for compounds that may be used for the manufacture of a medicament for the treatment of cholesterol associated conditions or diseases. In a preferred embodiment of the invention it was an object of the invention to provide for compounds that lower serum cholesterol and/or increase High Density lipoproteins (HDL) and/or decrease Low Density Lipoproteins (LDL). It was also an object of the invention to provide for compounds that may be used for the treatment of lipid disorders including hypercholesterolemia, atherosclerosis, Alzheimer's disease, skin disorders, obesity and diabetes.

### SUMMARY OF THE INVENTION

The present invention provides, *inter alia,* novel LXR nuclear receptor protein binding compounds according to the general formula (1) shown below. Said compounds are also binders of mammalian homologues of said receptor. Further the object of the invention was solved by providing for amongst the LXR nuclear receptor protein binding compounds according to the general formula (1) such compounds which act as agonists, antagonists or mixed agonists / antagonists of the human LXR receptor or a mammalian homologue thereof.

The invention provides for LXR agonists that may be used for the manufacture of a medicament for the treatment of cholesterol associated conditions or diseases. In a preferred embodiment of the invention it was an object of the invention to provide for compounds that lower serum cholesterol and/or increase High Density lipoproteins (HDL) and/or decrease Low Density Lipoproteins (LDL). It was also an object of the invention to provide for compounds that may be used for the treatment of lipid disorders including hypercholesterolemia, atherosclerosis, Alzheimer's disease, skin disorders, obesity and diabetes.

The foregoing merely summarizes certain aspects of the present invention and is not intended, nor should it be construed, to limit the invention in any manner. All patents and other publications recited herein are hereby incorporated by reference in their entirety.

### DETAILED DESCRIPTION OF THE PREFERRED EMBODIMENTS

The invention provides for a compound according to the following formula (1), or pharmaceutical acceptable salts or solvates thereof, hereinafter also referred to as the "compounds according to the invention" including particular and preferred embodiments thereof, wherein 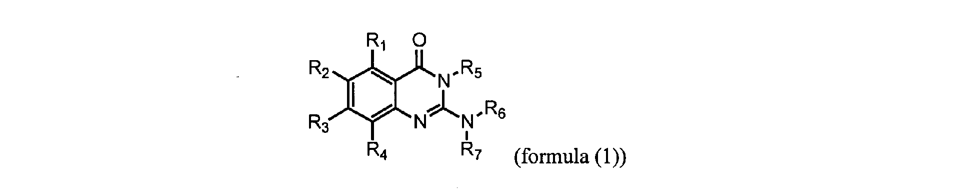 R₁, R₂, R₃ and/or R₄, is independently from each other selected from H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, wherein the phenyl is substituted or unsubstituted, such that, for example, a biphenyl results. R₅ is H, C₁ to C₈ alkyl, C₁ to C₈ substituted alkyl, C₇ to C₁₂ alkylphenyl or C₇ to C₁₂ substituted phenylalkyl, R₆ is H, C₁ to C₈ alkyl, C₁ to C₈ substituted alkyl, C₇ to C₁₂ alkylphenyl or C₇ to C₁₂ substituted phenylalkyl, R₇ is H, C₁ to C₈ alkyl, C₁ to C₈ substituted alkyl, C₇ to C₁₂ alkylphenyl or C₇ to C₁₂ substituted phenylalkyl, R₆ and R₇ may be taken together with nitrogen to form a heterocycle or substituted heterocycle or a heteroaryl or substituted heteroaryl ring.

The compounds of the invention can also exist as solvates and hydrates. Thus, these compounds may crystallize with, for example, waters of hydration, or one, a number of, or any fraction thereof of molecules of the mother liquor solvent. The solvates and hydrates of such compounds are included within the scope of this invention.

The symbol "H" denotes a hydrogen atom.

The term "C₁ to C₇ acyl" encompasses groups such as formyl, acetyl, propionyl, butyryl, pentanoyl, pivaloyl, hexanoyl, heptanoyl, benzoyl and the like. Preferred acyl groups are acetyl and benzoyl.

The term "C₁ to C₇ substituted acyl" denotes the acyl group substituted by one or more, and preferably one or two, halogen, hydroxy, protected hydroxy, oxo, protected oxo, cyclohexyl, naphthyl, amino, protected amino, (monosubstituted)amino, protected (monosubstituted)amino, (disubstituted)amino, guanidino, heterocyclic ring, substituted heterocyclic ring, imidazolyl, indolyl, pyrrolidinyl, C₁ to C₇ alkoxy, C₁ to C₇ acyl, C₁ to C₇ acyloxy, nitro, C₁ to C₆ alkyl ester, carboxy, protected carboxy, carbamoyl, carboxamide, protected carboxamide, N-(C₁ to C₆ alkyl)carboxamide, protected N-(C₁ to C₆ alkyl)carboxamide, N,N-di(C₁ to C₆ alkyl)carboxamide, cyano, methylsulfonylamino, thiol, C₁ to C₄ alkylthio or C₁ to C₄ alkylsulfonyl groups. The substituted acyl groups may be substituted once or more, and preferably once or twice, with the same or with different substituents.

The term "substituted phenyl" specifies a phenyl group substituted with one or more, and preferably one or two, moieties chosen from the groups consisting of halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, wherein the phenyl is substituted or unsubstituted, such that, for example, a biphenyl results.

Examples of the term "substituted phenyl" includes a mono- or di(halo)phenyl group such as 2, 3 or 4-chlorophenyl, 2,6-dichlorophenyl, 2,5-dichlorophenyl, 3,4-dichlorophenyl, 2, 3 or 4-bromophenyl, 3,4-dibromophenyl, 3-chloro-4-fluorophenyl, 2, 3 or 4-fluorophenyl and the like; a mono or di(hydroxy)phenyl group such as 2, 3 or 4-hydroxyphenyl, 2,4-dihydroxyphenyl, the protected-hydroxy derivatives thereof and the like; a nitrophenyl group such as 2, 3 or 4-nitrophenyl; a cyanophenyl group, for example, 2, 3 or 4-cyanophenyl; a mono- or di(alkyl)phenyl group such as 2, 3 or 4-methylphenyl, 2,4-dimethylphenyl, 2, 3 or 4-(iso-propyl)phenyl, 2, 3 or 4-ethylphenyl, 2, 3 or 4-(n-propyl)phenyl and the like; a mono or di(alkoxyl)phenyl group, for example, 2,6-dimethoxyphenyl, 2, 3 or 4-methoxyphenyl, 2, 3 or 4-ethoxyphenyl, 2, 3 or 4-(isopropoxy)phenyl, 2, 3 or 4-(t-butoxy)phenyl, 3-ethoxy-4-methoxyphenyl and the like; 2, 3 or 4-trifluoromethylphenyl; a mono- or dicarboxyphenyl or (protected carboxy)phenyl group such as 2, 3 or 4-carboxyphenyl or 2,4-di(protected carboxy)phenyl; a mono-or di(hydroxymethyl)phenyl or (protected hydroxymethyl)phenyl such as 2, 3, or 4-(protected hydroxymethyl)phenyl or 3,4-di(hydroxymethyl)phenyl; a mono- or di(aminomethyl)phenyl or (protected aminomethyl)phenyl such as 2, 3 or 4-(aminomethyl)phenyl or 2,4-(protected aminomethyl)phenyl; or a mono- or di(N-(methylsulfonylamino))phenyl such as 2, 3 or 4-(N-(methylsulfonylamino))phenyl. Also, the term "substituted phenyl" represents disubstituted phenyl groups wherein the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy 4-chlorophenyl and the like.

The term "heteroaryl" means a heterocyclic aromatic derivative which is a five-membered or six-membered ring system having from 1 to 4 heteroatoms, such as oxygen, sulfur and/or nitrogen, in particular nitrogen, either alone or in conjunction with sulfur or oxygen ring atoms.

Examples of heteroaryls include pyridinyl, pyrimidinyl, and pyrazinyl, pyridazinyl, pyrrolo, furano, thiopheno, oxazolo, isoxazolo, phthalimido, thiazolo and the like.

The term "substituted heteroaryl" means the above-described heteroaryl is substituted with, for example, one or more, and preferably one or two, substituents which are the same or different which substituents can be halogen, hydroxy, protected hydroxy, cyano, nitro, C₁ to C₆ alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino or N-(phenylsulfonyl)amino groups.

The term "substituted naphthyl" specifies a naphthyl group substituted with one or more, and preferably one or two, moieties either on the same ring or on different rings chosen from the groups consisting of halogen, hydroxy, protected hydroxy, cyano, nitro, C₁ to C₆ alkyl, C₁ to C₇ alkoxy, C₁ to C₇ acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino or N-(phenylsulfonyl)amino.

Examples of the term "substituted naphthyl" includes a mono or di(halo)naphthyl group such as 1, 2, 3, 4, 5, 6, 7 or 8-chloronaphthyl, 2, 6-dichloronaphthyl, 2, 5-dichloronaphthyl, 3, 4-dichloronaphthyl, 1, 2, 3, 4, 5, 6, 7 or 8-bromonaphthyl, 3, 4-dibromonaphthyl, 3-chloro-4-fluoronaphthyl, 1, 2, 3, 4, 5, 6, 7 or 8-fluoronaphthyl and the like; a mono or di(hydroxy)naphthyl group such as 1, 2, 3, 4, 5, 6, 7 or 8-hydroxynaphthyl, 2, 4-dihydroxynaphthyl, the protected-hydroxy derivatives thereof and the like; a nitronaphthyl group such as 3- or 4-nitronaphthyl; a cyanonaphthyl group, for example, 1, 2, 3, 4, 5, 6, 7 or 8-cyanonaphthyl; a mono- or di(alkyl)naphthyl group such as 2, 3, 4, 5, 6, 7 or 8-methylnaphthyl, 1, 2, 4-dimethylnaphthyl, 1, 2, 3, 4, 5, 6, 7 or 8-(isopropyl)naphthyl, 1, 2, 3, 4, 5, 6, 7 or 8-ethylnaphthyl, 1, 2, 3, 4, 5, 6, 7 or 8-(n-propyl)naphthyl and the like; a mono or di(alkoxy)naphthyl group, for example, 2, 6-dimethoxynaphthyl, 1, 2, 3, 4, 5, 6, 7 or 8-methoxynaphthyl, 1, 2, 3, 4, 5, 6, 7 or 8-ethoxynaphthyl, 1, 2, 3, 4, 5, 6, 7 or 8-(isopropoxy)naphthyl, 1, 2, 3, 4, 5, 6, 7 or 8-(t-butoxy)naphthyl, 3-ethoxy-4-methoxynaphthyl and the like; 1, 2, 3, 4, 5, 6, 7 or 8-trifluoromethylnaphthyl; a mono- or di-carboxynaphthyl or (protected carboxy)naphthyl group such as 1, 2, 3, 4, 5, 6, 7 or 8-carboxynaphthyl or 2, 4-di(-protected carboxy)naphthyl; a mono-or di(hydroxymethyl)naphthyl or (protected hydroxymethyl)naphthyl such as 1, 2, 3, 4, 5, 6, 7 or 8-(protected hydroxymethyl)naphthyl or 3, 4-di(hydroxymethyl)naphthyl; a mono- or di(amino)naphthyl or (protected amino)naphthyl such as 1, 2, 3, 4, 5, 6, 7 or 8-(amino)naphthyl or 2, 4-(protected amino)-naphthyl, a mono- or di(aminomethyl)naphthyl or (protected aminomethyl)naphthyl such as 2, 3, or 4-(aminomethyl)naphthyl or 2, 4-(protected aminomethyl)-naphthyl; or a mono- or di-(N-methylsulfonylamino) naphthyl such as 1, 2, 3, 4, 5, 6, 7 or 8-(N-methylsulfonylamino)naphthyl. Also, the term "substituted naphthyl" represents disubstituted naphthyl groups wherein the substituents are different, for example, 3-methyl-4-hydroxynaphth-1-yl, 3-chloro-4-hydroxynaphth-2-yl, 2-methoxy-4-bromonaphth-1-yl, 4-ethyl-2-hydroxynaphth-1-yl, 3-hydroxy-4-nitronaphth-2-yl, 2-hydroxy-4-chloronaphth-1-yl, 2-methoxy-7-bromonaphth-1-yl, 4-ethyl-5-hydroxynaphth-2-yl, 3-hydroxy-8-nitronaphth-2-yl, 2-hydroxy-5-chloronaphth-1-yl and the like.

The term "C₁ to C₈ alkyl" denotes such radicals as methyl, ethyl, n-propyl, isopropyl, n-butyl, iso-butyl, sec-butyl, tert-butyl, amyl, tert-amyl, hexyl , n-heptyl, 2-heptyl, 3-heptyl, 4-heptyl, 2-methyl-lhexyl, 2-methyl-2hexyl, 2-methyl-3-hexyl, n-octyl and the like.

Examples of the above substituted alkyl groups include the 2-oxo-prop-1-yl, 3-oxo-but-1-yl, cyanomethyl, nitromethyl, chloromethyl, hydroxymethyl, tetrahydropyranyloxymethyl, trityloxymethyl, propionyloxymethyl, amino, methylamino, aminomethyl, dimethylamino, carboxymethyl, allyloxycarbonylmethyl, allyloxycarbonylaminomethyl, methoxymethyl, ethoxymethyl, t-butoxymethyl, acetoxymethyl, chloromethyl, bromomethyl, iodomethyl, trifluoromethyl, 6-hydroxyhexyl, 2,4-dichloro(n-butyl), 2-aminopropyl, 1-chloroethyl, 2-chloroethyl, 1- bromoethyl, 2-chloroethyl, 1-fluoroethyl, 2-fluoroethyl, 1- iodoethyl, 2-iodoethyl, 1-chloropropyl, 2-chloropropyl, 3- chloropropyl, 1-bromopropyl, 2-bromopropyl, 3-bromopropyl, 1-fluoropropyl, 2-fluoropropyl, 3-fluoropropyl, 1- iodopropyl, 2-iodopropyl, 3-iodopropyl, 2-aminoethyl, 1- aminoethyl, N-benzoyl-2-aminoethyl, N-acetyl-2-aminoethyl, N-benzoyl-1-aminoethyl, N-acetyl-1-aminoethyl and the like.

The term "C₁ to C₈ substituted alkyl" denotes that the above C₁ to C₈ alkyl groups are substituted by one or more, and preferably one or two, halogen, hydroxy, protected hydroxy, oxo, protected oxo, C₃ to C₇ cycloalkyl, naphthyl, amino, protected amino, (monosubstituted)amino, protected (monosubstituted)amino, (disubstituted)amino, guanidino, protected guanidino, heterocyclic ring, substituted heterocyclic ring, imidazolyl, indolyl, pyrrolidinyl, C₁ to C₇ alkoxy, C₁ to C₇ acyl, C₁ to C₇ acyloxy, nitro, carboxy, protected carboxy, carbamoyl, carboxamide, protected carboxamide, N-(C₁ to C₆ alkyl)carboxamide, protected N-(C₁ to C₆ alkyl)carboxamide, N,N-di(C₁ to C₆ alkyl)carboxamide, cyano, methylsulfonylamino, thiol, C₁ to C₄ alkylthio or C₁ to C₄ alkylsulfonyl groups. The substituted alkyl groups may be substituted once or more, and preferably once or twice, with the same or with different substituents.

The term "C₇ to C₁₂ phenylalkyl" denotes a C₁ to C₆ alkyl group substituted at any position by a phenyl, substituted phenyl, heteroaryl or substituted heteroaryl. Examples of such a group include benzyl, 2-phenylethyl, 3-phenyl(n-propyl), 4-phenylhexyl, 3-phenyl(n-amyl), 3-phenyl(sec-butyl) and the like. Preferred C₇ to C₁₂ phenylalkyl groups are the benzyl and the phenylethyl groups.

The term "C₇ to C₁₂ substituted phenylalkyl" denotes a C₇ to C₁₂ phenylalkyl group substituted on the C₁ to C₆ alkyl portion with one or more, and preferably one or two, groups chosen from halogen, hydroxy, protected hydroxy, oxo, protected oxo, amino, protected amino, (monosubstituted)amino, protected (monosubstituted)amino, (disubstituted)amino, guanidino, protected guanidino, heterocyclic ring, substituted heterocyclic ring, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, nitro, carboxy, protected carboxy, carbamoyl, carboxamide, protected carboxamide, N-(C₁ to C₆ alkyl)carboxamide, protected N-(C₁ to C₆ alkyl)carboxamide, N, N-(C₁ to C₆ dialkyl)carboxamide, cyano, N-(C₁ to C₆ alkylsulfonyl)amino, thiol, C₁ to C₄ alkylthio, C₁ to C₄ alkylsulfonyl groups; and/or the phenyl group may be substituted with one or more, and preferably one or two, substituents chosen from halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl) carboxamide, protected N-(C₁ to C₆ alkyl) carboxamide, N, N-di(C₁ to C₆ alkyl)carboxamide, trifluoromethyl, N-((C₁ to C₆ alkyl)sulfonyl)amino, N-(phenylsulfonyl)amino, cyclic C₂ to C₇ alkylene or a phenyl group, substituted or unsubstituted, for a resulting biphenyl group. The substituted alkyl or phenyl groups may be substituted with one or more, and preferably one or two, substituents which can be the same or different.

Examples of the term "C₇ to C₁₂ substituted phenylalkyl" include groups such as 2-phenyl-1-chloroethyl, 2-(4-methoxyphenyl)ethyl, 4-(2,6-dihydroxy phenyl)n-hexyl, 2-(5-cyano-3-methoxyphenyl)n-pentyl, 3-(2,6-dimethylphenyl)n-propyl, 4-chloro-3-aminobenzyl, 6-(4-methoxyphenyl)-3-carboxy(n-hexyl), 5-(4-aminomethylphenyl)- 3-(aminomethyl)n-pentyl, 5-phenyl-3-oxo-n-pent-1-yl and the like.

As outlined above R₆ and R₇ may be taken together with nitrogen to form a heterocycle or substituted heterocycle of the following kind aziridine, azetidine, pyrrolidine, 3-methylpyrrolidine, 3-aminopyrrolidine, 3-hydroxypyrrolidine, pyrazolidine, imidazolidine, piperidine, 2-methylpiperidine, piperazine, morpholine, azepine, tetrahydroisoquinoline

The term "heterocycle" or "heterocyclic ring" denotes optionally substituted five-membered to eight-membered rings that have 1 to 4 heteroatoms, such as oxygen, sulfur and/or nitrogen, in particular nitrogen, either alone or in conjunction with sulfur or oxygen ring atoms. These five-membered to eight-membered rings may be saturated, fully unsaturated or partially unsaturated, with fully saturated rings being preferred. Preferred heterocyclic rings include morpholino, piperidinyl, piperazinyl, 2-amino-imidazoyl, tetrahydrofurano, pyrrolo, tetrahydrothiophen-yl, hexylmethyleneimino and heptylmethyleneimino.

The term "substituted heterocycle" or "substituted heterocyclic ring" means the above-described heterocyclic ring is substituted with, for example, one or more, and preferably one or two, substituents which are the same or different which substituents can be halogen, hydroxy, protected hydroxy, cyano, nitro, C₁ to C₁₂ alkyl, C₁ to C₁₂ alkoxy, C₁ to C₁₂ substituted alkoxy, C₁ to C₁₂ acyl, C₁ to C₁₂ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino carboxamide, protected carboxamide, N-(C₁ to C₁₂ alkyl)carboxamide, protected N-(C₁ to C₁₂ alkyl)carboxamide, N, N-di(C₁ to C₁₂ alkyl)carboxamide, trifluoromethyl, N-((C₁ to C₁₂ alkyl)sulfonyl)amino, N-(phenylsulfonyl)amino, heterocycle or substituted heterocycle groups.

The term "C₁ to C₈ alkoxy" as used herein denotes groups such as methoxy, ethoxy, n-propoxy, isopropoxy, n-butoxy, t-butoxy and like groups. A preferred alkoxy is methoxy. The term "C₁ to C₈ substituted alkoxy" means the alkyl portion of the alkoxy can be substituted in the same manner as in relation to C₁ to C₈ substituted alkyl.

The term "C₁ to C₈ aminoacyl" encompasses groups such as formyl, acetyl, propionyl, butyryl, pentanoyl, pivaloyl, hexanoyl, heptanoyl, octanoyl, benzoyl and the like.

The term "C₁ to C₈ substituted aminoacyl" denotes the acyl group substituted by one or more, and preferably one or two, halogen, hydroxy, protected hydroxy, oxo, protected oxo, cyclohexyl, naphthyl, amino, protected amino, (monosubstituted)amino, protected (monosubstituted)amino, (disubstituted)amino, guanidino, heterocyclic ring, substituted heterocyclic ring, imidazolyl, indolyl, pyrrolidinyl, C₁ to C₁₂ alkoxy, C₁ to C₁₂ acyl, C₁ to C₁₂ acyloxy, nitro, C₁ to C₁₂ alkyl ester, carboxy, protected carboxy, carbamoyl, carboxamide, protected carboxamide, N-(C₁ to C₁₂ alkyl)carboxamide, protected N-(C₁ to C₁₂ alkyl)carboxamide, N,N-di(C₁ to C₁₂ alkyl)carboxamide, cyano, methylsulfonylamino, thiol, C₁ to C₁₀ alkylthio or C₁ to C₁₀ alkylsulfonyl groups. The substituted acyl groups may be substituted once or more, and preferably once or twice, with the same or with different substituents.

Examples of C₁ to C₈ substituted acyl groups include 4-phenylbutyroyl, 3-phenylbutyroyl, 3-phenylpropanoyl, 2- cyclohexanylacetyl, cyclohexanecarbonyl, 2-furanoyl and 3-dimethylaminobenzoyl.

This invention provides a pharmaceutical composition comprising an effective amount of a compound according to the invention. Such compounds can be administered by various routes, for example oral, subcutaneous, intramuscular, intravenous or intracerebral. The preferred route of administration would be oral at daily doses of the compound for adult human treatment of about 0.01 -5000 mg, preferably 1-1500 mg per day. The appropriate dose may be administered in a single dose or as divided doses presented at appropriate intervals for example as two, three four or more subdoses per day.

For preparing pharmaceutical compositions containing compounds of the invention, inert, pharmaceutically acceptable carriers are used. The pharmaceutical carrier can be either solid or liquid. Solid form preparations include, for example, powders, tablets, dispersible granules, capsules, cachets, and suppositories.

A solid carrier can be one or more substances which can also act as diluents, flavoring agents, solubilizers, lubricants, suspending agents, binders, or tablet disintegrating agents; it can also be an encapsulating material.

In powders, the carrier is generally a finely divided solid which is in a mixture with the finely divided active component. In tablets, the active compound is mixed with the carrier having the necessary binding properties in suitable proportions and compacted in the shape and size desired.

For preparing pharmaceutical composition in the form of suppositories, a low-melting wax such as a mixture of fatty acid glycerides and cocoa butter is first melted and the active ingredient is dispersed therein by, for example, stirring. The molten homogeneous mixture is then poured into convenient-sized molds and allowed to cool and solidify.

Powders and tablets preferably contain between about 5% to about 70% by weight of the active ingredient. Suitable carriers include, for example, magnesium carbonate, magnesium stearate, talc, lactose, sugar, pectin, dextrin, starch, tragacanth, methyl cellulose, sodium carboxymethyl cellulose, a low-melting wax, cocoa butter and the like.

The pharmaceutical compositions can include the formulation of the active compound with encapsulating material as a carrier providing a capsule in which the active component (with or without other carriers) is surrounded by a carrier, which is thus in association with it. In a similar manner, cachets are also included. Tablets, powders, cachets, and capsules can be used as solid dosage forms suitable for oral administration.

Liquid pharmaceutical compositions include, for example, solutions suitable for oral or parenteral administration, or suspensions, and emulsions suitable for oral administration. Sterile water solutions of the active component or sterile solutions of the active component in solvents comprising water, ethanol, or propylene glycol are examples of liquid compositions suitable for parenteral administration.

Sterile solutions can be prepared by dissolving the active component in the desired solvent system, and then passing the resulting solution through a membrane filter to sterilize it or, alternatively, by dissolving the sterile compound in a previously sterilized solvent under sterile conditions.

In one embodiment of the present invention a compound is claimed according to formula (1) above, or pharmaceutical acceptable salts or solvates thereof, wherein R₁, R₂, R₃, R₄, is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, R₅ is H, C₁ to C₈ alkyl, C₁ to C₈ substituted alkyl, C₇ to C₁₂ alkylphenyl or C₇ to C₁₂ substituted phenylalkyl, R₆ and R₇ may be taken together with nitrogen to form the heterocycle according to formula (2),

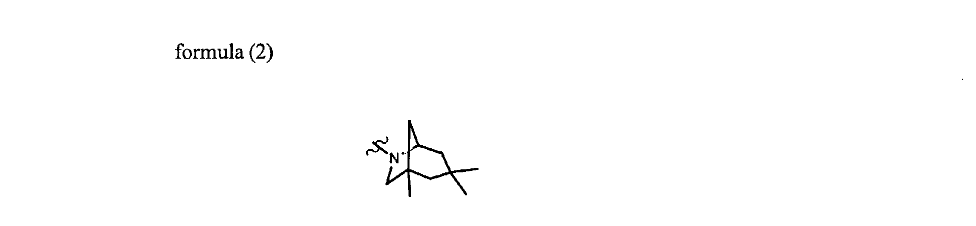

In a preferred embodiment of the invention a compound is provided, or pharmaceutical acceptable salts or solvates thereof, wherein R₁, R₂, R₃, R₄, is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, R₅ is H, C₁ to C₈ alkyl, C₁ to C₈ substituted alkyl, R₆ and R₇ may be taken together with nitrogen to form the heterocycle according to formula (2) shown above.

A particularly preferred compound which may act as agonist of LXR is shown in formula (6) below. The inventors have been able to demonstrate that the compound according to formula (3) has a low effective concentration at LXR with an EC₅₀ of 0.5 µM wherein the EC₅₀ reflects the half-maximal effective concentration, and which is higher than the EC₅₀ of 0.015 µM for the published LXR agonist TO901317 (J. Schultz et al., Genes Dev. 14, 2831-2838, 2000)

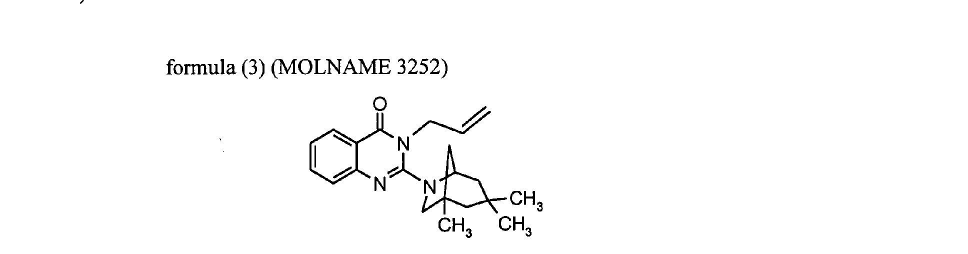

The inventors have also found the compounds according to formula (4, 5 and 6) (shown below) to be active as agonist of the LXR human nuclear receptor (see figures for details).

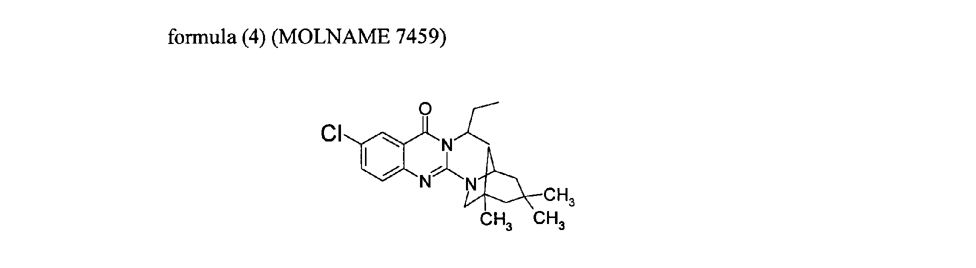

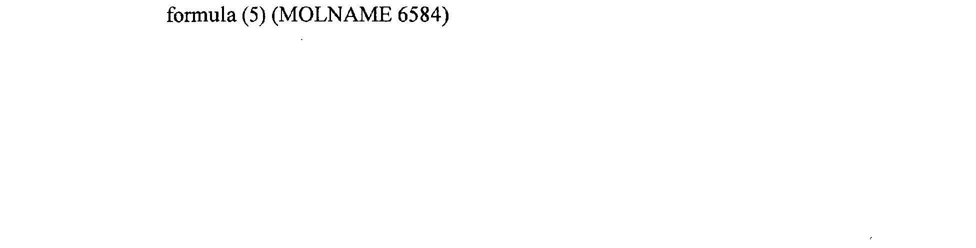

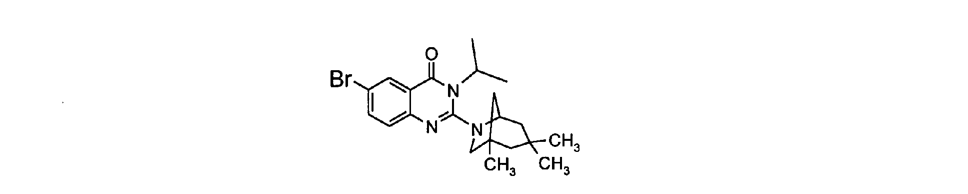

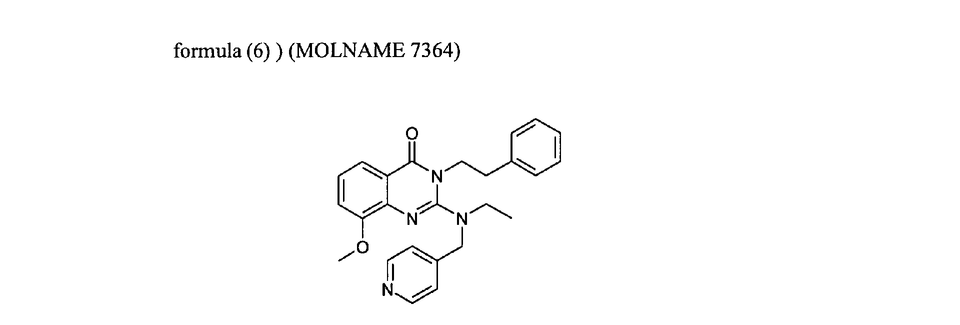

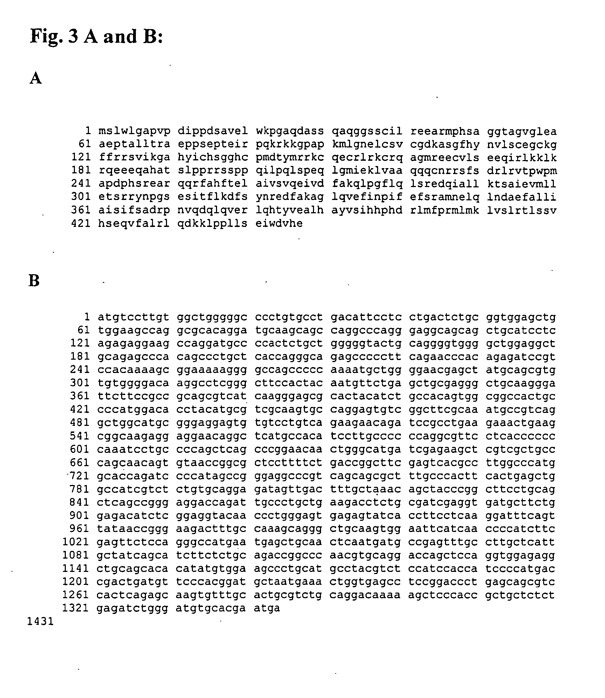

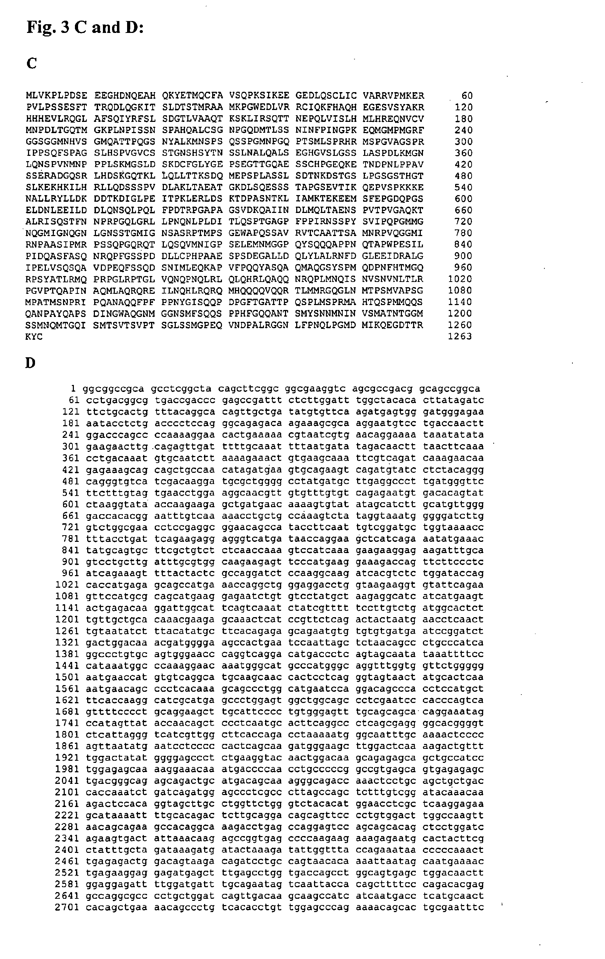

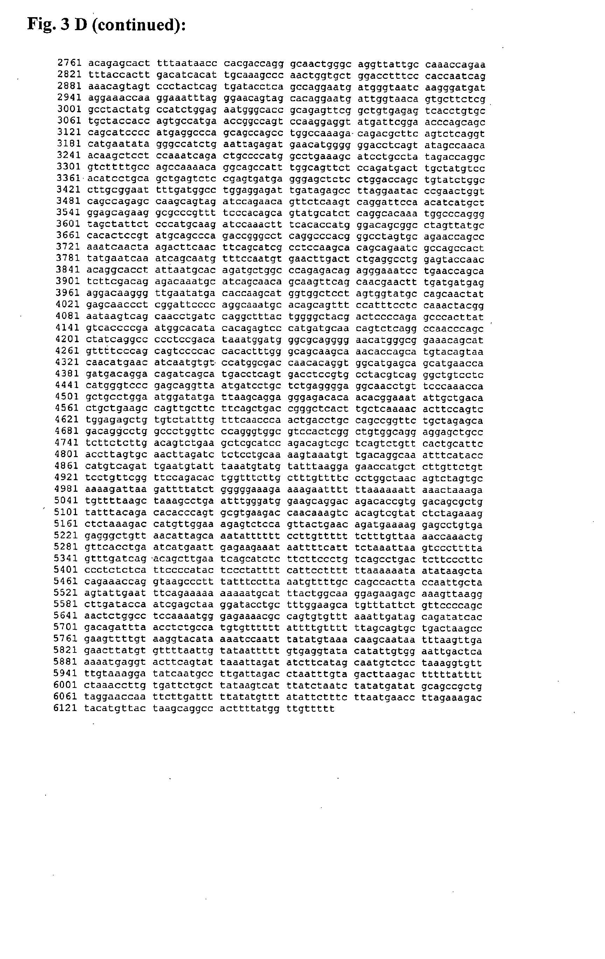

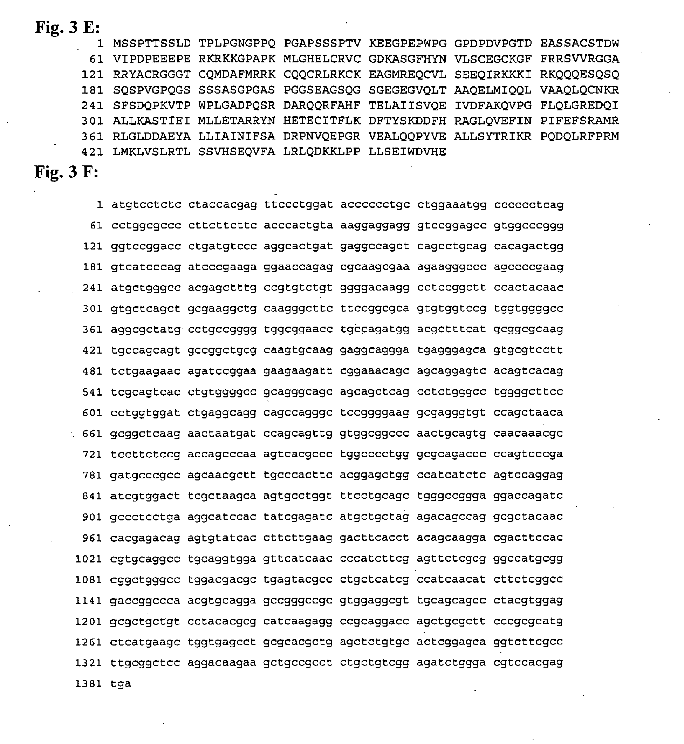

In particular the invention relates to a compound as described above wherein said compounds is capable of binding the LXR receptor protein or a portion thereof according to SEQ ID NO. 1 (Fig. 3 A to F) or a mammalian homologue thereof. The claimed compound can bind to the LXR receptor protein or a portion thereof in a mixture comprising 10-200 ng of LXR receptor protein, a fusion protein containing LXR or a portion thereof, preferably the ligand binding domain, fused to a Tag, 5-100 mM Tris /HCl at pH 6,8-8,3 ; 60-1000 mM KCl; 0-20 mM MgCl2; 100-1000ng/µl BSA in a total volume of preferably about 25 µl.).

A mammalian receptor protein homologue of the protein according to SEQ ID NO. 1 as used herein is a protein that performs substantially the same task as LXR does in humans and shares at least 40% sequence identity at the amino acid level, preferably over 50 % sequence identity at the amino acid level more preferably over 65 % sequence identity at the amino acid level, even more preferably over 75 % sequence identity at the amino acid level and most preferably over 85 % sequence identity at the amino acid level.

The invention in particular concerns a method for prevention or treatment of a LXR receptor protein or LXR receptor protein homologue mediated disease or condition in a mammal comprising administration of a therapeutically effective amount of a compound according to the invention wherein the prevention or treatment is directly or indirectly accomplished through the binding of a compound according to the invention to the LXR receptor protein or to the LXR receptor protein homologue.

The term mediated herein means that the physiological pathway in which the LXR receptor protein acts is either directly or indirectly involved in the disease or condition to be treated or prevented. In the case where it is indirectly involved it could be that, e.g. modulating the activity of LXR by a compound according to the invention influences a parameter which has a beneficial effect on a disease or a condition. One such example is that modulation of LXR activity leads to decreased levels of serum cholesterol or certain lipoproteins which in turn have a beneficial effect on the prevention and treatment of atherosclerosis. Herein a condition is a physiological or phenotypic state which is desirably altered. One such example would be obesity which is not necessarily medically harmful but nonetheless a non desirable phenotypic condition. In a preferred embodiment of the invention the method for prevention or treatment of a LXR receptor protein mediated disease or condition is applied to a human. This may be male or female.

Pharmaceutical compositions generally are administered in an amount effective for treatment or prophylaxis of a specific condition or conditions. Initial dosing in human is accompanied by clinical monitoring of symptoms, such symptoms for the selected condition. In general, the compositions are administered in an amount of active agent of at least about 100 µg/kg body weight. In most cases they will be administered in one or more doses in an amount not in excess of about 20 mg/kg body weight per day. Preferably, in most cases, doses is from about 100 µg/kg to about 5 mg/kg body weight, daily.

For administration particularly to mammals, and particularly humans, it is expected that the daily dosage level of active agent will be 0,1 mg/kg to 10 mg/kg and typically around 1 mg/kg.

By "therapeutically effective amount" is meant a symptom- alleviating or symptom -reducing amount, a cholesterol-reducing amount, a cholesterol absorption blocking amount, a protein and/or carbohydrate digestion-blocking amount and/or a de novo cholesterol biosynthesisblocking amount of a compound according to the invention.

Likewise, the invention concerns a method of treating in mammal a disease which is correlated with abnormal cholesterol, triglyceride, or bile acid levels or deposits comprising administering to a mammal in need of such treatment a therapeutically effective amount of a compound according to the invention.

Accordingly, the compounds according to the invention may also be used as a method of prevention or treatment of mammalian atherosclerosis, gallstone disease, lipid disorders, Alzheimer's disease, skin disorders, obesity or cardiovascular disorders such as coronary heart disease or stroke.

The invention further concerns a method of blocking in a mammal the cholesterol absorption in the intestine in need of such blocking comprising administering to a mammal in need of such treatment a therapeutically effective amount of a compound according to the invention. The invention may also be used to treat obesity in humans.

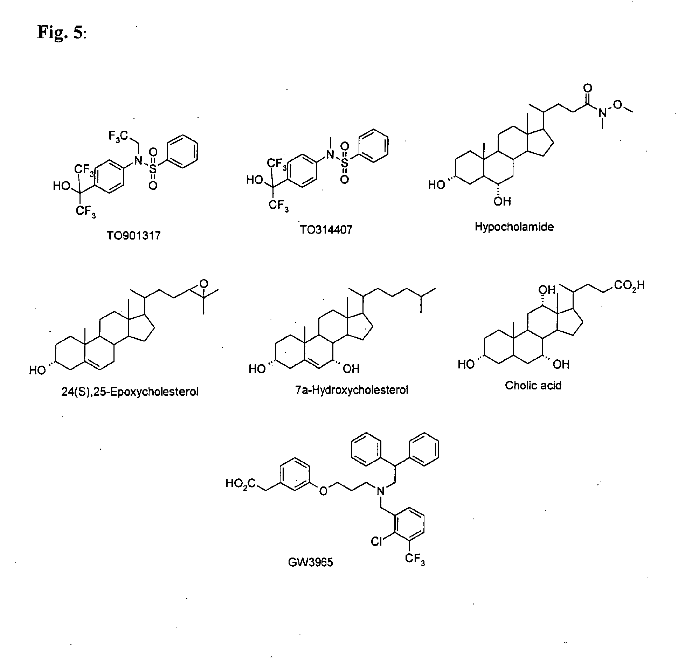

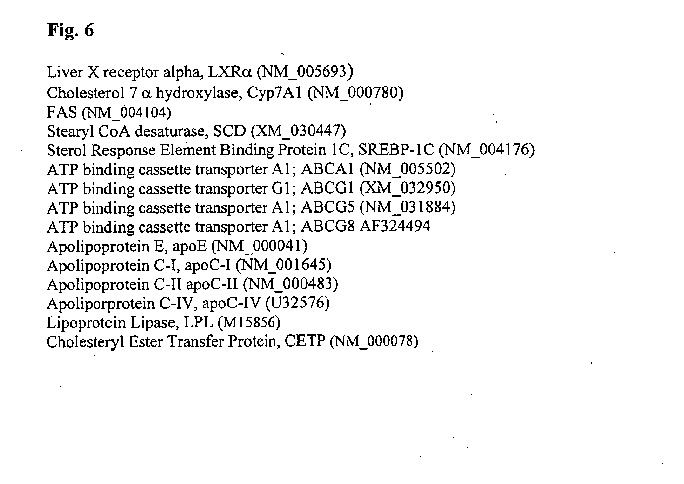

The Liver X Receptor alpha is a prototypical type 2 nuclear receptor meaning that it activates genes upon binding to the promoter region of target genes in a heterodimeric fashion with Retinoid X Receptor. The relevant physiological ligands of LXR are oxysterols The present compounds according to the invention have been demonstrated to have a high binding efficacy (binding coefficients measured as EC50 in the range 100 nM to 1500 nM) as well as agonistic and / or antagonistic properties. Consequently they may be applied to regulate genes that participate in bile acid, cholesterol and fatty acid homeostasis as well as other downstream regulated genes. Examples of such genes are but are not limited to lipid absorption, cholesterol biosynthesis, cholesterol transport or binding, bile acid transport or binding, proteolysis, amino acid metabolism, glucose biosynthesis, protein translation, electron transport, and hepatic fatty acid metabolism. LXR often functions in vivo as a heterodimer with the Retinoid X Receptor. Published LXR agonists such as the Tularik compound "TO901317" (See figure 5) are known to influence the regulation of various liver genes. Genes found to be regulated by TO901317 can be found in figure 6. Thus, the invention also concerns a method of modulating a gene whose expression is regulated by the LXR receptor in a mammal comprising administration of a therapeutically effective amount of a compound according to the invention to said mammal.

A number of direct and indirect LXR target genes have been described whose regulated expression contribute to cholesterol homeostasis and lipogenesis. In this respect the direct regulation of Cyp7A, which was shown to be a direct target gene of LXR at least in the rodent lineage is an important aspect of cholesterol removal by increased metabolism of bile acids (Lehmann et al., J Biol.Chem. 272 (6) 3137-3140; 1007). Gupta et al. (Biochem. Biophys Res.Com, 293; 338-343, 2002) showed that LXR α regulation of Cyp7A is dominant over FXR inhibitory effects on Cyp7A transcription.

A key transcription factor that was also shown to be a direct target gene for the LXR receptor is SREBP-1C (Repa et al., Genes and Development, 14:2819-2830; 2000: Yoshikawa et al.; Mol.Cell.Biol.21 (9) 2991-3000, 2001). SREBP-1C itself activates transcription of genes involved in cholesterol and fatty acid synthesis in liver but also other mammalian tissues. Some of the SREBP1c target genes involved in lipogenesis like FAS and SCD have shown to be additionally direct targets of the LXR receptors (Joseph et al.; J Biol Chem. 2002 Mar 29;277(13):11019-25; Liang et al., J Biol Chem. 2002 Mar 15;277(11):9520-8.).

Another gene that has been shown to be directly regulated by LXRs is the LPL gene, that codes for a key enzyme that is responsible for the hydrolysis of triglycerides in circulating lipoprotein, releasing free fatty acids to peripheral tissues. (Zhang et al. J Biol Chem. 2001 Nov 16;276(46):43018-24.) This enzyme is believed to promote uptake of HDL cholesterol in liver, thereby promoting reverse cholesterol transport. A similar functional involvement in HDL clearance is described for the CETP gene product that facilitated the transfer of HDL cholesterol esters from plasma to the liver. LXR response elements were found in the CETP promoter and direct activation of this gene by LXR was demonstrated (Luo and Tall; J Clin Invest. 2000 Feb;105(4):513-20.).

The regulated transport of cholesterol through biological membranes is an important mechanism in order to maintain cholesterol homeostasis. A pivotal role in these processes in multiple tissues like e.g. macrophages and intestinal mucosa cells is maintained by the ATPbinding cassette transporter proteins (ABC). ABCA1 and ABCG1 were identified as direct LXR target genes (Costet et al.; J Biol Chem. 2000 Sep 8;275(36):28240-5) that mediate cholesterol efflux and prevent thereby e.g. generation of artherogenic plaques in macrophages (Singaraja et al. J Clin Invest. 2002 Jul;110(1):35-42). Other ABC transporters like ABCG5 and ABCG8 , primarily expressed in hepatocytes and enterocytes have also been reported to be directly responsive to LXR agonists ( Repa et al., J Biol Chem. 2002 May 24;277(21):18793-800. Kennedy et al., J Biol Chem. 2001 Oct 19;276(42):39438-47) and mediate the secretion of sterols from the liver and efflux of dietary sterols from the gut.

Apolipoproteins E, C-I, C-II, and C-IV, that fulfill important roles in lipoprotein/lipid homeostasis have also been shown to be direct targets of the LXR receptor ( Laffitte et al., Proc Natl Acad Sci U S A. 2001 Jan 16;98(2):507-12; Mak et al.; J Biol Chem. 2002 May 24 [epub ahead of print]). These proteins have been found to be crucial components of chylomicrons, VLDL, IDL, and HDL and are among other things associated with hypertriglyceridemia and arteriosclerosis.

Recently the LXRα itself was shown to be regulated by both LXR receptors in human cell types including macrophages suggesting an autoregulatory amplification event in the response to LXR ligands which could e.g. lead to an enhanced stimulation of LXR target genes like e.g. ABCA1 (Bolten et al.; Mol Endocrinol. 2002 Mar;16(3):506-14.; Laffitte et al., Mol Cell Biol. 2001 Nov;21(22):7558-68; Whitney et al.; J Biol Chem. 2001 Nov 23;276(47):43509-15).

Besides the important function of LXR receptors in tissues like liver and macrophages it has recently been reported that that stimulation of epidermal differentiation is mediated by Liver X receptors in murine epidermis. Differentiation maker genes like involucrin, loricin and profilaggrin have been shown to be upregulated upon LXR ligand treatment (Kömüves et al.; J Invest Dermatol. 2002 Jan;118(1):25-34.).

Another recent report describes the regulation of cholesterol homeostasis (primarily the regulation of ABCA1, ABCG1 and SREBP-1C) by the LXR receptors in the central nervous system suggesting that LXRs may prove beneficial in the treatment of CNS diseases such as Alzheimer's and Niemann-Pick disease that are known to be accompanied by dysregulation of cholesterol balance (Whitney et al.; Mol Endocrinol. 2002 Jun;16(6):1378-85).

Therefore one important embodiment the invention concerns are methods that enhances or suppresses amongst other today yet unknown LXR target genes the above mentioned genes and the associated biological processes and pathways through LXR compounds that are subject of this invention.

The compounds according to the invention may be used as medicaments, in particular for the manufacture of a medicament for the prevention or treatment of a LXR receptor protein or LXR receptor protein homologue mediated disease or condition in a mammal wherein the prevention or treatment is directly or indirectly accomplished through the binding of the compound according to the invention to the LXR receptor protein or LXR receptor protein homologue. These pharmaceutical compositions contain 0,1 % to 99,5 % of the compound according to the invention, more particularly 0,5 % to 90 % of the compound according to the invention in combination with a pharmaceutically acceptable carrier.

The invention concerns also the use of a compound according to the invention for the manufacture of a medicament for the prevention or treatment of a LXR receptor protein mediated disease or condition wherein the mammal described above is a human. The medicament may be used for regulating the cholesterol transport system , for regulating levels of cholesterol, triglyceride, and/or bile acid in a mammal preferentially a human by activating the LXR receptor. The medicament may be used for the treatment of atherosclerosis, gallstone disease, lipid disorders, Alzherimer's disease, skin disorders, obesity or a cardiovascular disorder.

The further concerns the use of a compound according to the invention for the manufacture of a medicament capable for blocking in a mammal, preferentially a human the cholesterol absorption in the intestine. Further the claimed compound may be used for the manufacture of a medicament for treating obesity in humans and for modulating a gene whose expression is regulated by the LXR receptor (see details above and figures).

The present invention shall now be further illustrated based on the following examples without being limited thereto. In the accompanying sequence protocol and the figures:
SEQ ID NO. 1 shows protein sequence of the LRX alpha protein a portion of which was used for cloning as described in the examples,
SEQ ID NO. 2 shows the mRNA sequence of the LRX alpha protein,
SEQ ID NO. 3 shows the protein sequence of TIF2 (Acc. No: XM_011633 RefSeq DB),
SEQ ID NO. 4 shows the respective mRNA sequence corresponding to the TIF2 protein,
SEQ ID NO 5 shows the protein sequence of the LXR beta protein a portion of which was used for cloning as described in examples,
SEQ ID NO 6 shows the mRNA sequence of the LXR beta protein,
SEQ ID NO 7 shows the sequence of primer (a) used in Example 1
SEQ ID NO 8 shows the sequence of primer (b) used in Example 1.

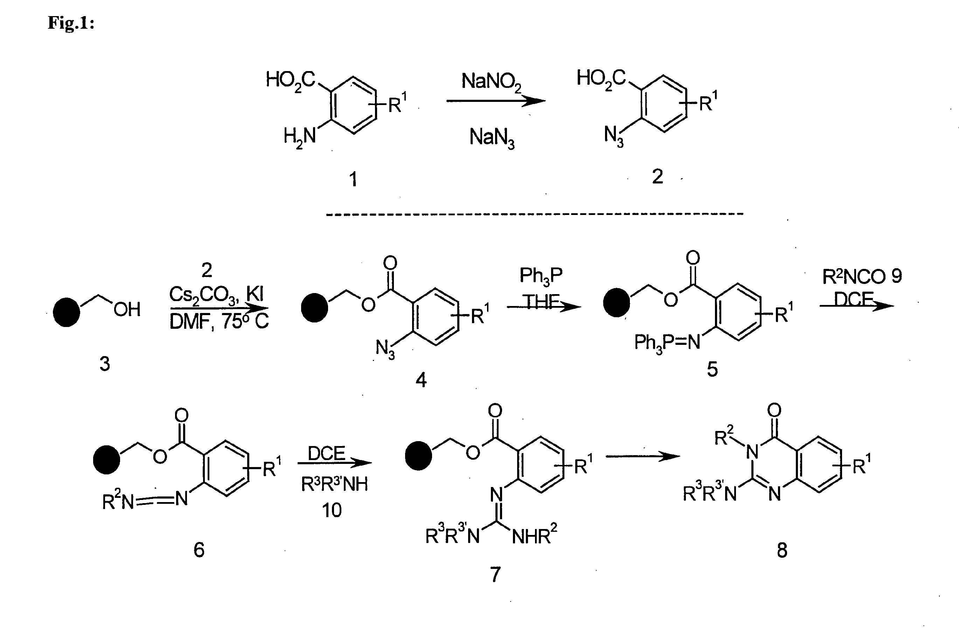

Fig. 1 shows the synthesis of the compounds according to the invention as also described in Example 2.

Fig. 2 shows the measurement parameters employed by the Wallace VICTOR2V™ Multilabel Counter which was used for measuring the EC₅₀ values

Fig. 3 A shows SEQ ID NO. 1 which is the protein sequence of the LRX alpha protein a portion of which was used for cloning as described in the examples . Figure 3 B shows SEQ ID NO. 2 which is the mRNA sequence of the LRX alpha protein. Figure 3 C shows SEQ ID NO. 3 which is the protein sequence of TIF2 (Acc. No: XM_011633 RefSeq DB), Figure 3 D shows SEQ ID NO. 4 which is the respective mRNA sequence corresponding to the TIF2 protein. Figure 3 E shows SEQ ID NO 5 which is the protein sequence of the LXR beta protein a portion of which was used for cloning as described in examples. Figure 3 F shows SEQ ID NO 6 which is the mRNA sequence of the LXR beta protein.

Fig. 4 shows the internal molecular name used by the applicant (MOLNAME) as well as the corresponding structures of preferred compounds according to the invention. The figure further shows their respective EC₅₀ values (EC50 AVG) as established according to the experiment 1 in multiple experiments (see above), as well as their respective average efficacy (% activity relative to 22-(R)-hydroxycholesterol control agonist).

Figure 5 shows various known LXR ligands. It is apparent from their structures that the inventors have identified novel compounds which are structurally not related to these known ligands.

Figure 6 shows various genes that have been found to be regulated through binding of an LXR agonist to the LXR protein.

Figure 7 shows a dose-dependent transactivation (EC50 ∼ 3 µM) by LN0000007465 of the luciferase reporter gene via LXR alpha.

Figure 8 shows (A) Analysis of mRNA content of the indicated genes in total RNA isolated from THP-1 cells treated for 24 hours with 2, 10 or 25µM of LN0000006500 or 10 µM of the Tularik compound (T0901317). (B) Analysis of mRNA content fo the indicated genes in total RNA from HepG2 cells treated for 24 hours with 2, 10 or 25 µM of LN0000006500 or 10 µM of the Tularik compound (T0901317).

Figure 9 shows the dose dependent transactivation by LN0000006500 of the pFR-luc reporter gene in CHO cells via Gal4 LBD-fusion constructs derived from LXRa- or LXRb. Concentrations of the compound administered (µM) and RLU's determined from extracts of cells are indicated.

Figure 10 shows the analysis of total cholesterol from supernatants of cultivated THP-1 cells incubated without or with ApoA1 and ApoA1 plus 10µM of the compounds Tularik (T0901317) or LN0000006500, LN0000006662, LN0000006671 or LN0000006672 as indicated.

### EXAMPLES

### EXAMPLE 1:

In vitro screening for compounds which influence LXR binding to coactivators.
For screening purposes a GST and 6 x His fusion of the LBD (from amino acids 155 of hLXRalpha to 447) of human LXRalpha was constructed by first cloning a Gateway cassette (Invitrogen) in frame into the Sma I site of the pAGGHLT Polylinker (Pharmingen). Then a PCR fragment specifically amplified from human liver cDNA was cloned into the resulting pACGHLT-GW following the manufacturers instructions for Gateway cloning (Invitrogen) to yield pACGHLT-GW-hLXRalphaLBD.

Primers used for amplification were: primer (a)
GGGGACAAGTTTGTACAAAAAAGCAGGCTCGCTTCGCAAATGCCGTCAG (SEQ ID
NO 7), and primer (b)
GGGGACCACTTTGTACAAGAAAGCTGGGTCCCCTTCTCAGTCTGTTCCACTT (SEQ
ID NO 8).

100 % sequence integrity of all recombinant products was verified by sequencing. Recombinant Baculovirus was constructed from pACGHLT-GW-hLXRalphaLBD using the Pharmingen Baculovirus Expression vector system according to instructions of the manufacturer. Monolayer cultures of SF9 cells were infected by the virus as recommended by Pharmingen or 200ml cultures of 1 x10⁶ cells/ml grown in 2 liter Erlenmeyer flasks on an orbital shaker at 30 rpm were infected by 10ml of same virus stock. In both cases cells were harvested 3 days after infection. All cell growth was performed in Gibco SF900 II with Glutamine (Invitrogen) medium without serum supplementation at 28°C. Since SF9 cells contain significant amounts of endogenous GST, purification was performed via His and not via GST affinity chromatography. To this end instructions of Pharmingen for purification of recombinant His tagged proteins from SF9 cells were followed with the following modifications: All detergents were omitted from the buffers and cells were lysed on ice by 5 subsequent sonication pulses using a sonicator needle at maximum power.

All eluates were dialyzed against 20 mM Tris/HCl pH 6,8, 300 mM KCl; 5 mM MgCl₂; 1 mM DTT; 0,2 mM PMSF; 10% Glycerol. A typical dialyzed eluate fraction contained the fusion protein at a purity of more than 80%. Total protein concentration was 0,1-0,3 mg/ml.

For E. coli expression of a NR coactivator, pDest17-hTif2BD expressing a NR interaction domain from amino acids 548-878 of human Tif2 (Acc. No: XM_011633 RefSeq) tagged by 6 N-terminal His residues was constructed. Therefore, a PCR fragment specifically amplified from human liver cDNA was subcloned into pDest 17 (Invitrogen) following the manufacturers instructions for Gateway cloning (Invitrogen).Primers used for Amplification were: primer (a)

GGGGACAAGTTTGTACAAAAAAGCAGGCTCGTTAGGGTCATCGTTGGCTTCACC

and primer (b)
GGGGACCACTTTGTACAAGAAAGCTGGGTCTCAAAGTTGCCCTGGTCGTGGGTTA
.

For E. coli expression plasmid DNA was transformed into chemically competent E. coli BL21 (Invitrogen, USA) and cells were grown to an OD600 of 0.4-0.7 before expression was induced by addition of 0,5 mM IPTG according instructions of the manufacturer (Invitrogen). After induction for 8 hours at 30°C cells were harvested by centrifugation for 10 minutes at 5000 x g. Fusion proteins were affinity purified using Ni-NTA Agarose (QIAGEN) according to the instructions of the manufacturer. Recombinant Tif2 construct was dialyzed against 20 mM Tris/HCL pH 7.9; 60 mM KCl; 5 mM MgCl₂; 1 mM DTT, 0,2 mM PMSF; 10% glycerol. A typical dialyzed eluate fraction contained the fusion protein at a purity of more than 80%. Total protein concentration was 0,1-0,3 mg/ml.

The TIF2 fragment was subsequently biotinylated by addition of 5-40µl/ml Tif2 fraction of a Biotinamidocaproate N-Hydroxysuccinimide-ester (Sigma) solution (20 mg/ml in DMSO). Overhead rotating samples were incubated for 2 hours at room temperature. Unincorporated label was then separated using G25 Gel filtration chromatography (Pharmacia Biotech, Sweden). Protein containing fractions from the column were pooled and tested for activity in the assay as described below.

For screening of compound libraries as provided for by the methods shown below in the examples for substances which influence the LXR/Tif 2 interaction, the Perkin Elmer LANCE technology was applied. This method relies on the binding dependent energy transfer from a donor to an acceptor fluorophore attached to the binding partners of interest. For ease of handling and reduction of background from compound fluorescence LANCE technology makes use of generic fluorophore labels and time resoved detection (for detailed description see Hemmilä I, Blomberg K and Hurskainen P, Time-resolved resonance energy transfer (TR-FRET) principle in LANCE, Abstract of Papers Presented at the 3 rd Annual Conference of the Society for Biomolecular Screening, Sep., California (1997) )

For screening, 20-200 ng of biotinylated Tif 2 fragment and 10-200 ng of GST-LXR fragment were combined with 0.5-2 nM LANCE Eu-(W1024) labelled anti-GST antibody (Perkin Elmer) and 0,1-0,5µg of highly fluorescent APC-labelled streptavidin (Perkin Elmer, AD0059) in the presence of 50µM of individual compounds to be screened in a total volume of 25 µl of 20 mM Tris /HCl pH 6,8; 300 mM KCl; 5 mM MgCl2; 100-1000 ng/µl/ BSA DMSO content of the samples was kept below 4%. Samples were incubated for a minimum of 60 minutes in the dark at room temperature in FIA-Plates black 384well med. binding (Greiner).

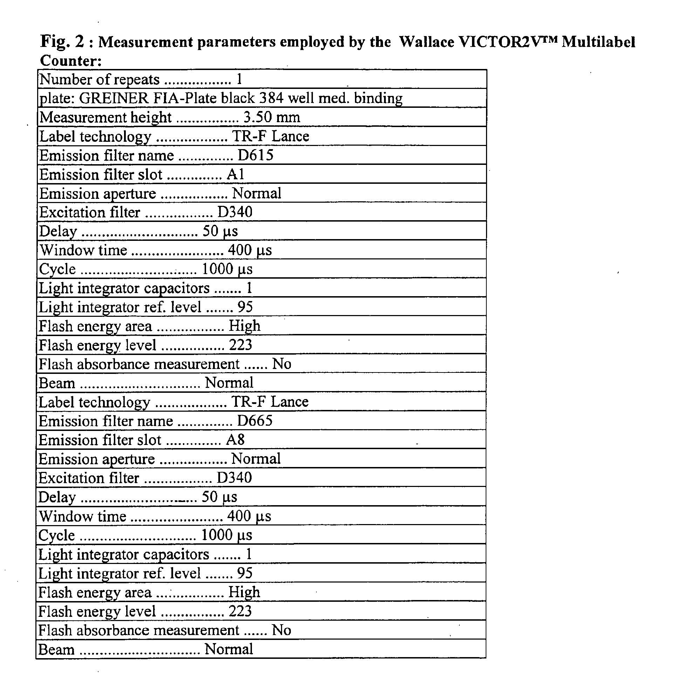

The LANCE signal was detected by a Perkin Elmer VICTOR2V™ Multilabel Counter applying the detection parameters listed in Fig. 2. The results were visualized by plotting the ratio between the emitted light at 665 nm and at 615 nm. For every batch of recombinant proteins amount of proteins, including BSA and labeling reagents giving the most sensitive detection of hits was determined individually by analysis of dose response curves for 22R Hydroxycholesterol and TO 901317

### EXAMPLE 2:

Experimental procedure for the preparation of the compounds according to the invention.

### o-AZIDOBENZOIC ACID SYNTHESIS (2)

The anthranilic acid (1, 1 eq., 0.5-1 M) was suspended in 6 M HCl, containing enough AcOH (0-20% dependent upon the anthranilic acid) to facilitate dissolution of the anthranilic acid and/or the intermediate diazonium salt, and cooled to 0 °C. NaNO₂ (1.1 eq., 1.3-2.5 M) dissolved in H₂O was added to the anthranilic acid solution at a rate such that the temperature of the reaction solution remained below 5 °C. The resulting homogeneous solution of the diazonium salt was slowly filtered through a sintered glass funnel into a solution of NaN₃ (1.1 eq., 0.7-1.1 M) and NaOAc (12 eq.) in H₂O. The reaction mixture was stirred/shaken for 30-60 min following cessation of vigorous N₂ evolution. Following acidification of the reaction mixture to pH 1 with concentrated HCl, the mixture was cooled to 0 °C to encourage complete precipitation of the *o*-azidobenzoic acid. The precipitate was collected by filtration and washed with 6 M HCl (2x) and H₂O (2x). The *o*-azidobenzoic acid product (2) was dried in *vacuo* (500 mtorr, 30 °C).

### ACYLATION OF HYDROXYMETHYL RESIN (4)

To hydroxymethyl resin (1.0 eq., 1.3 mmol/g) and the *o*-azidobenzoic acid (1, 2.5 eq.) was added DMF (to give 400 mM *o*-azidobenzoic acid ,1), CsCO₃ (2.0 eq.) and KI (2.0 eq.). Following agitation of the reaction mixture for 36-48 h, the resin-bound *o*-azidobenzoic acid (4) was washed with MeOH (2 cycles), CH₂Cl₂ (3 cycles), MeOH (3 cycles), DMF (3 cycles), MeOH (3 cycles) and CH₂Cl₂ (3 cycles), and dried *in vacuo.*

### AZA-WITTIG FORMATION (5)

To the resin-bound o-azidobenzoic acid (4,1.0 eq.) was added a solution of PPh₃ (THF, 500 mM, 5.0 eq.). After 6 h, the resin was washed with 3 cycles of the following: THF (3 cycles), toluene (3 cycles), CH₂Cl₂ (3 cycles) and hexanes (3 cycles). Followed by drying *in vacuo* to afford resin bound iminophosphorane (5)

### CARBODIIMIDE FORMATION (6)

To the resin-bound iminophosphorane (5, 1 eq.) was added isocyanate (9 , 5 eq., 450 mM) dissolved in ClCH₂CH₂Cl. The compounds were shaken at ambient temperature for 16 h, washed with 3 cycles of the following: THF (3 cycles), toluene (3 cycles), CH₂Cl₂ (3 cycles) and hexanes (3 cycles), and dried *in vacuo* to afford carbodiimide (6).

### GUANIDINE FORMATION / CYCLIZATION

To the carbodiimide functionalized resin (6) was added secondary amine (10, 0.6 eq., 500 mM) dissolved in ClCH₂CH₂Cl. The reaction mixture was heated to 50 °C in an incubator for 12-72 h to afford 2-aminoquinazoline (8).

All of the final products were analyzed by HPLC using mass and an Evaporative Light Scattering Detector (ELSD) detection to determine purity and identity.

One skilled in the art will be able to arrive at the compounds claimed herein making use of said protocol.

### EXAMPLE 3:

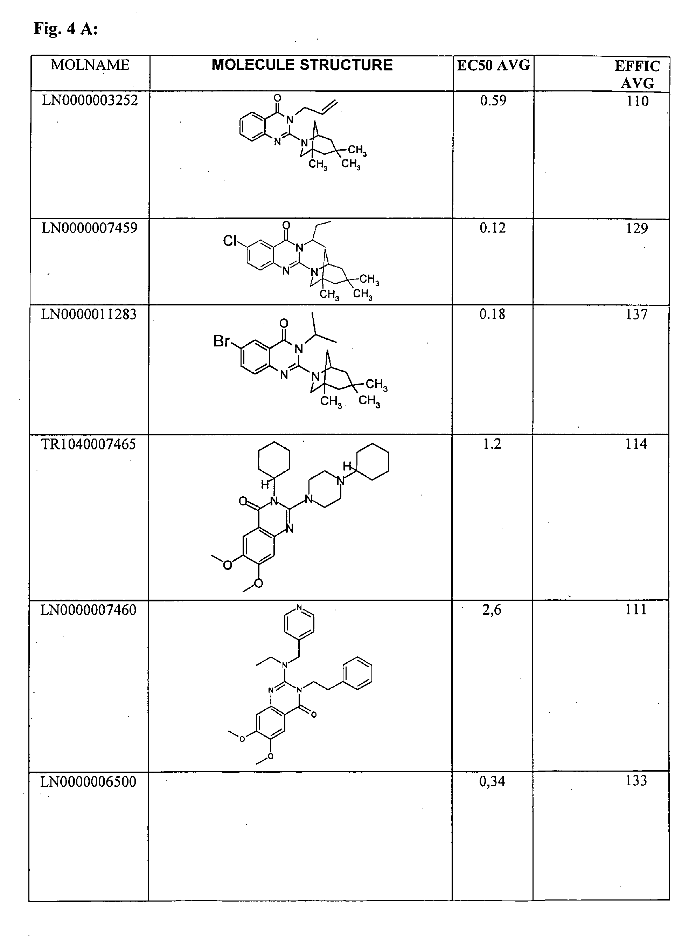

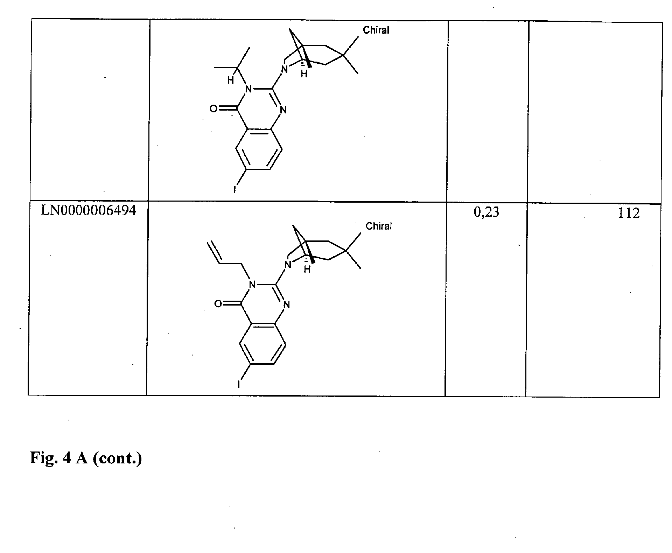

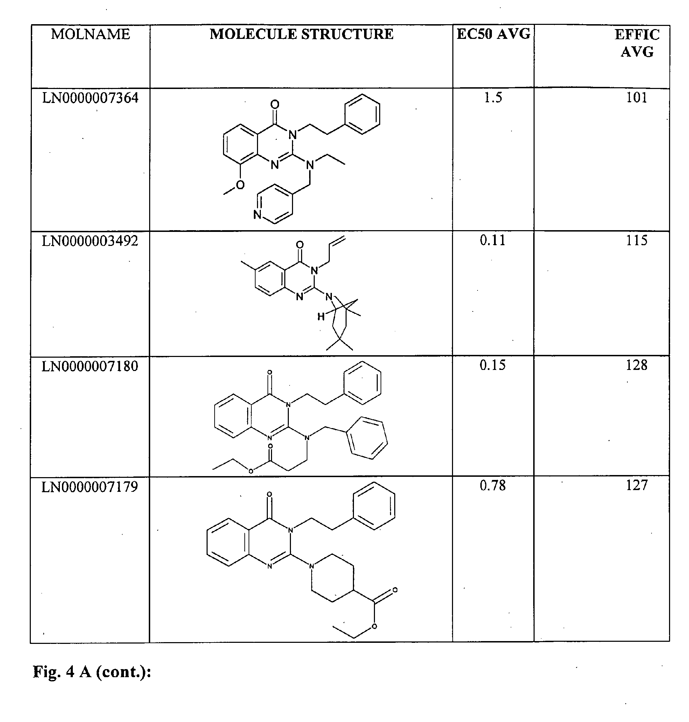

This example illustrates that a compound according to the invention (experiments shown were done with MOLSTRUCTURE LN 0000007465 (see figures 4 for structural formula)) can mediate transactivation of LXR mediated transcription in HEK293 cells.

HEK293 cells were grown in 48 well plates and co-transfected with the pTRexDest30 (Invitrogen) derivatives pTRexDest30-hLXRa, pTRexDest30-hRXR□ and the pGL2promoter (Promega) derivative pGL2promoter-LXRRE (each 300 ng of plasmid DNA). The full length human LXR (accession U68233) and the full length human RXRα (accession P19793) were cloned into the pTRexDest30 applying the manufacturer protocols for the Gateway™ system (Invitrogen).

The LXR response elements (LXRRE) were (upper case and underlined) 5' Ccctt**TGGTCActcaAGTTCA**agtgatgatagaattcggatcctt**TGGTCActcaAGTTCA**agtgA 3' (SEQ ID NO. 5) derived from the rat Cyp7a promoter (Laffite et al., 2001, PNAS 98,pp 507). Luciferase reporter activity was measured in triplicates from extracts of cells after incubating cells in culture medium (DMEM [Gibco-BRL] + 10% FCS [PAA laboratories]) for 16 hours (5% CO₂, 37°C) containing 0,5% DMSO (control) or 0,5% DMSO with increasing concentrations of LN0000007465.

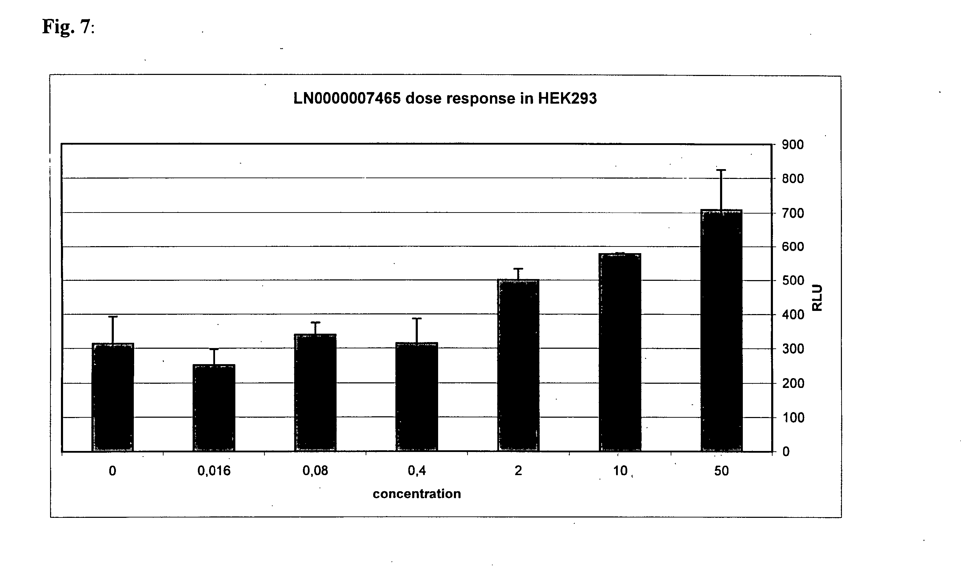

A dose-dependent transactivation (EC50 ∼ 3 µM) of the reporter gene by LXRa was observed (Fig. 7).

### Example 4:

This example shows that described compounds can increase the abundance of mRNA of target genes for the LXR proteins in THP-1 cells treated with TPA.

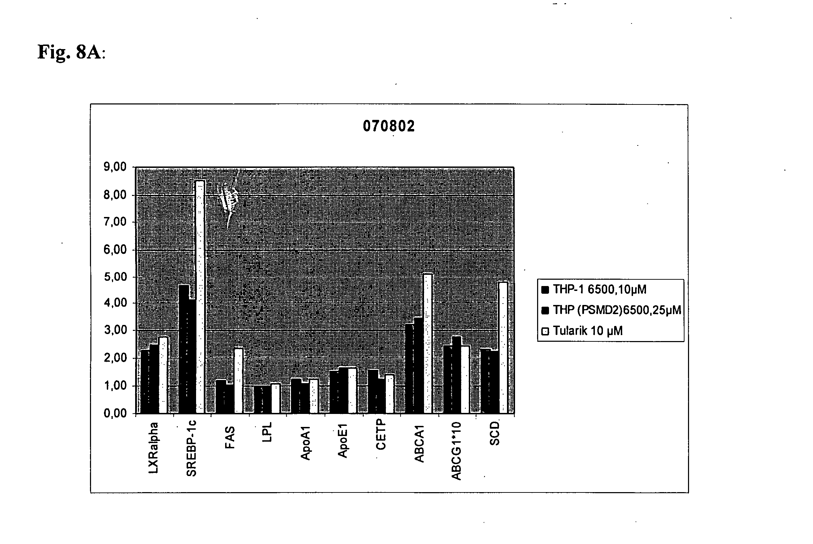

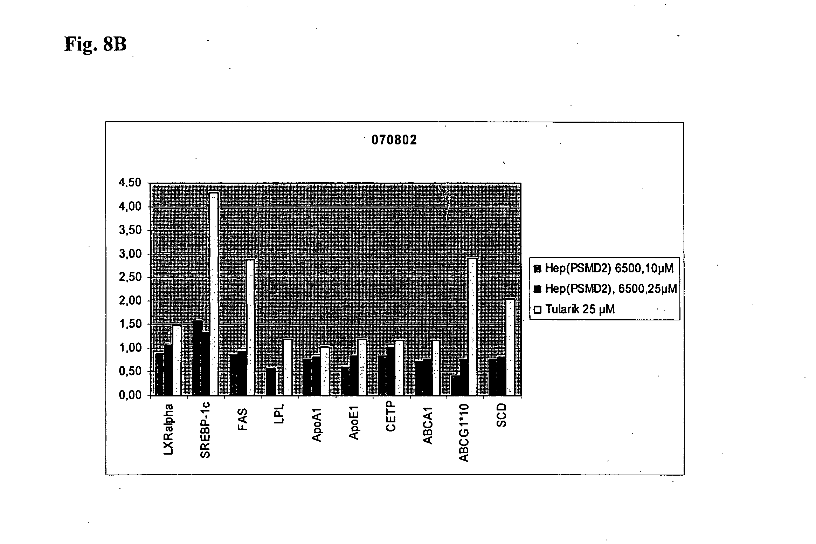

THP-1 ( 3x10⁵ cells per dish) cells were seeded in 24 well dishes in 3 ml modified RPMI-1640 medium (ATTC, Cat.No. 30-2001) containing 10%FCS (GIBCO) and 100nM TPA and cultivated at 37°C in 5% CO₂ for 48 hours. The medium was then removed and replaced with medium containing 10% charcoal/stripped FBS (Hyclone) and incubated with LN0000006500 at 2, 10 or 25 µM concentration or Tularik (T0901317) at 10 µM for 24 hours as indicated in Fig 8A as an example. HepG2 (4,5x10⁵ cells per dish) were seeded in 24 well dishes in 3 ml DMEM Medium containing 10%FCS (GIBCO) and cultivated at 37°C in 5% CO₂ for 48 hours as indicated in Fig 8B.

After incubation for 24 hours in presence of compound, total RNA was isolated from the cells using a Quiagen RNAeasy kit (Quiagen) according to the manufacturers protocol. The RNA was then reverse transcribed and analyzed by TaqMan Analysis using kits and equipment from Perkin-Elmer known to those knowledgeable in the field.

The fold change of mRNA abundancy of compound treated versus DMSO treated as a control is shown in Figure 8A and B for several analyzed target genes indicated in Figure 8Aand B.

### Example 5:

This example shows that described compounds can selectively enhance transcription mediated by the LBD's of the respective nuclear receptors LXRa and LXRb.

CHO cells (1x10⁵ cells 96well plate) were co-transfected (Lipofectamine 2000 GIBCO) with pFR-luc (Stratagene) as a reporter gene construct and pCMV-AD derivatives containing the LXRa or LXRb ligand binding domains, which were cloned via the gateway system (GIBCO) described in Example 1, in order to express Gal4DBD-LXRa or Gal4DBD-LXRb fusion proteins.

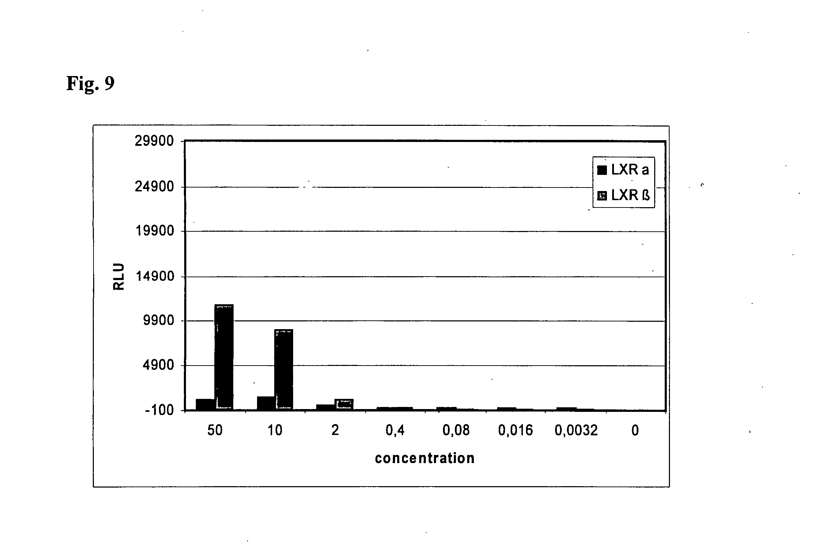

Cells were grown in DMEM containing 10%FCS at 37°C in 5% humidified CO2 for 16h in presence of 0,05% DMSO vehicle or 0,032 to 50 µM LN0000006500 in vehicle (as indicated in Fig.9). Luciferase activity was determined from aliquots of extracts prepared from cells following standard luciferase assay kits and protocols from Promega.

### Example 6:

This example shows that described compounds at 10 µM concentration for 24 hours can increase the reverse cholesterol transport in THP-1 cells that were treated with TPA.

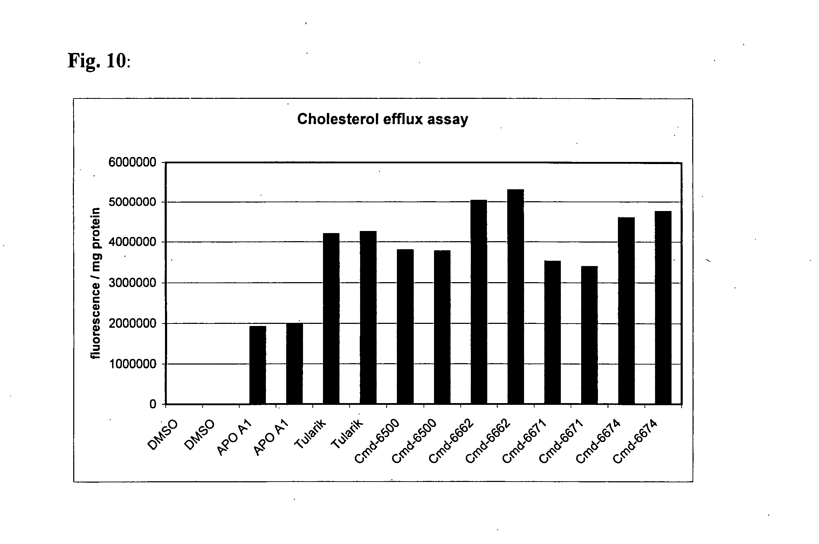

THP-1 (1x10⁶ cells per dish) cells were seeded in 6 well dishes in 3ml modified RPMI1640 medium ( ATTC, Cat.No. 30-2001) containing 10%FCS (GIBCO) and 100nM TPA and cultivated at 37°C in 5% CO₂ for 72 hours. The medium was then removed and replaced with fresh medium containing 100 nM TPA and 0,15 % BSA. After 24 h incubation the cells were washed in PBS and 1,5 ml of fresh medium containing either 0,1% DMSO alone or 0,1% DMSO together with 40µg/ml ApoA1 (Calbiochem) or 40µg/ml ApoA1 plus the in Fig. 10 as an example indicated compounds Tularik (T0901317), LN0000006500, LN0000006662, LN0000006671, LN0000006674 at 10 µM.

After incubation for 24 hours, total cholesterol was determined from cell supernatant in each of the wells using an enzymatic assay with fluorescence read-out for the determination of cholesterol (Amplex Red Cholesterol Assay Kit (A-12213). The fluorescence readout per mg of total protein content as determined from cells that were present in the respective well are shown in Figure 9 as an example.

## Claims

1. A compound of the formula (1), or pharmaceutical acceptable salts or solvates thereof according to formula (1) 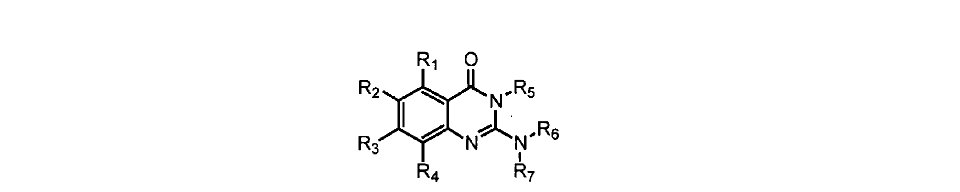 wherein:
R₁, R₂, R₃ and/or R₄, is independently from each other H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N-(phenylsulfonyl)amino or phenyl, wherein the phenyl is substituted or unsubstituted,
R₅ is H, C₁ to C₈ alkyl, C₁ to C₈ substituted alkyl, C₇ to C₁₂ alkylphenyl or C₇ to C₁₂ substituted phenylalkyl,
R₆ is H, C₁ to C₈ alkyl, C₁ to C₈ substituted alkyl, C₇ to C₁₂ alkylphenyl or C₇ to C₁₂ substituted phenylalkyl, and
R₇ is H, C₁ to C₈ alkyl, C₁ to C₈ substituted alkyl, C₇ to C₁₂ alkylphenyl or C₇ to C₁₂ substituted phenylalkyl.

2. A compound according to claim 1 wherein R₆ and R₇ are taken together with nitrogen to form a heterocycle or substituted heterocycle or a heteroaryl or substituted heteroaryl according to the following formula (2).

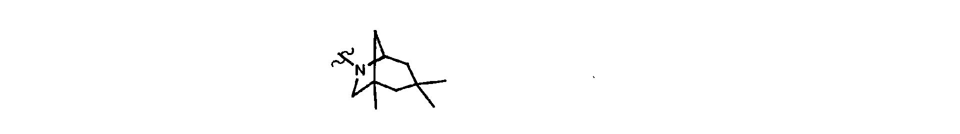

3. A compound according to claim 2, or pharmaceutical acceptable salts or solvates thereof, wherein:
R₁, R₂, R₃, R₄, is H, halogen, hydroxy, protected hydroxy, cyano, nitro, C₁ to C₆ alkyl, C₁ to C₆ substituted alkyl, C₁ to C₇ alkoxy, C₁ to C₇ substituted alkoxy, C₁ to C₇ acyl, C₁ to C₇ substituted acyl, C₁ to C₇ acyloxy, carboxy, protected carboxy, carboxymethyl, protected carboxymethyl, hydroxymethyl, protected hydroxymethyl, amino, protected amino, (monosubstituted)amino, protected (monosubstituted)amino, (disubstituted)amino, carboxamide, protected carboxamide, N-(C₁ to C₆ alkyl)carboxamide, protected N-(C₁ to C₆ alkyl)carboxamide, N, N-di(C₁ to C₆ alkyl)carboxamide, trifluoromethyl, N-((C₁ to C₆ alkyl)sulfonyl)amino, N- (phenylsulfonyl)amino or phenyl, and
R₅ is H, C₁ to C₈ alkyl, C₁ to C₈ substituted alkyl.

4. A compound according to claim 1 wherein R₆ and R₇ are taken together with nitrogen to form the heterocycle according to the following formula (3)

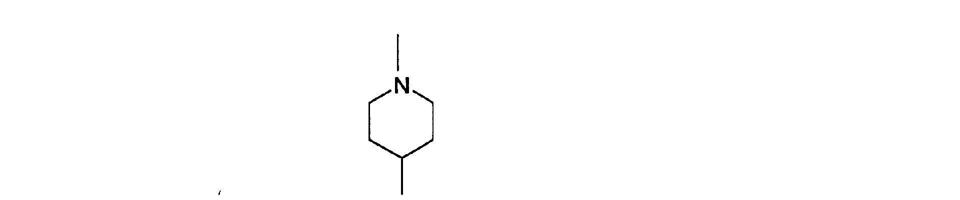

5. A compound according to any of claims 1 to 3 of the following formula (4)

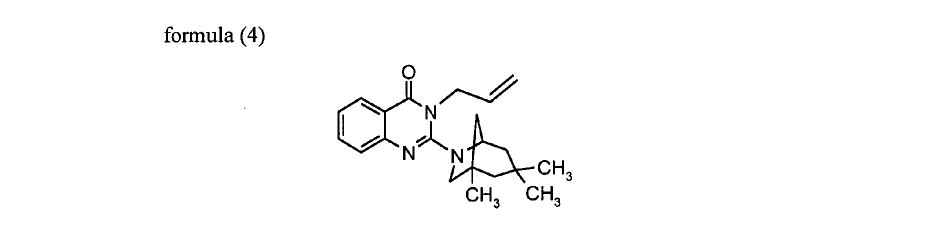

6. A compound according to any of claims 1 to 3 of the following formula (5)

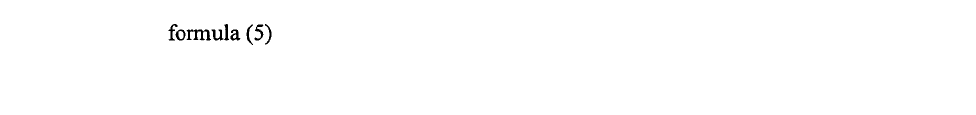

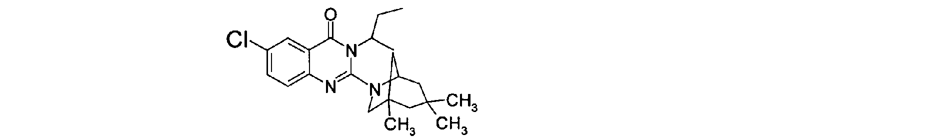

7. A compound according to any of claims 1 to 3 of the following formula (6)

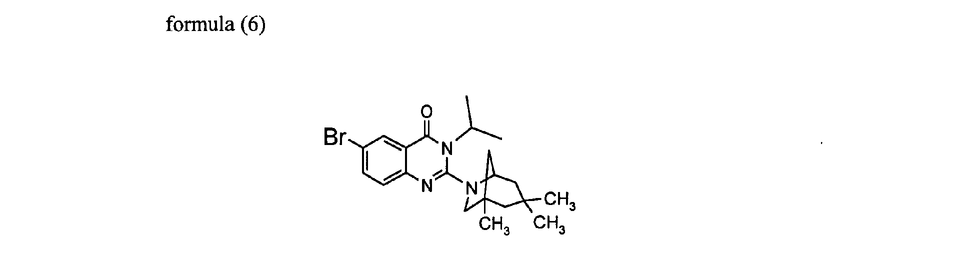

8. A compound according to any of claims 1 and 4 wherein R₆ and R₇ are taken together with nitrogen to form the heterocycle according to the following formula (7)

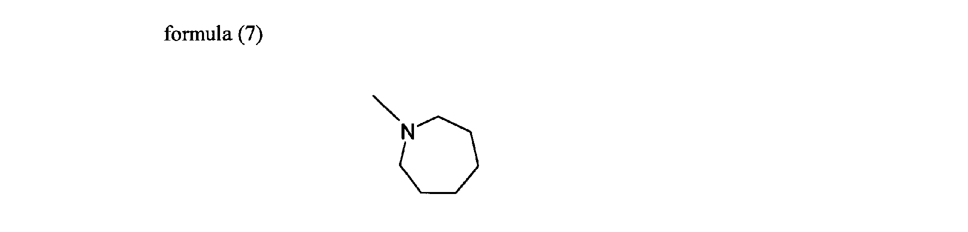

9. A compound according to claim 1 according to the following formula (8)

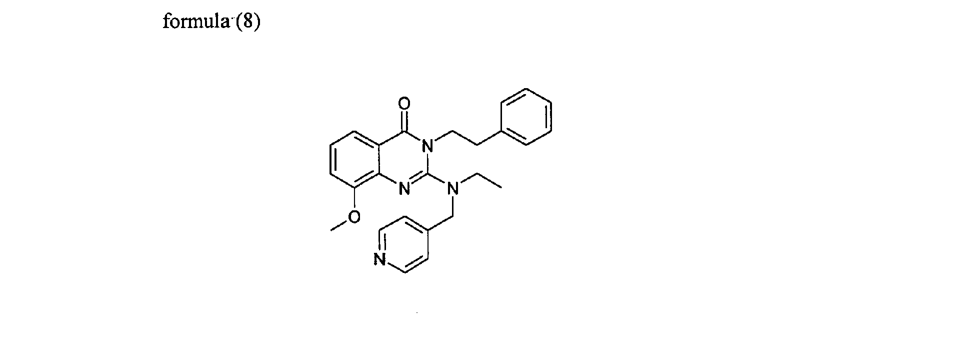

10. A compound according to claims 1 and 4 according to the following formula (8)

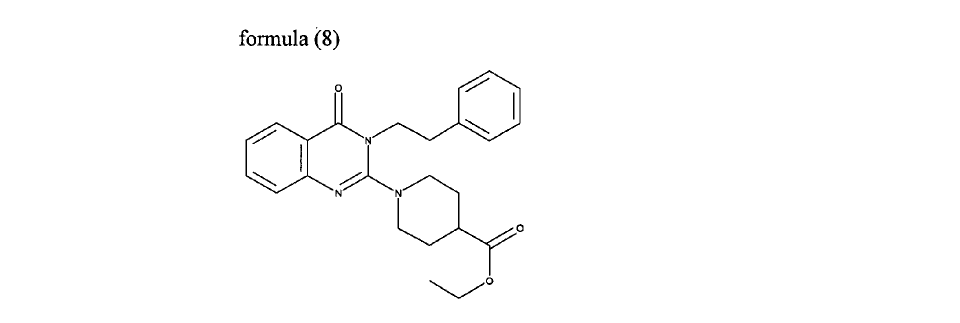

11. A compound according to any of claims 1 to 10 wherein said compound is capable of binding the NR1H3 receptor protein or a portion thereof according to SEQ ID NO. 1 or a mammalian homologue thereof.

12. A compound according to any of claims 1 to 10 wherein said compound is capable of binding the NR1H2 receptor protein or a portion thereof or a mammalian homologue thereof.

13. Use of a compound according to any of claims 1 to 12 as a medicament

14. A method for prevention or treatment of a NR1H3 and/or NR1H2 receptor protein mediated disease or NR1H3 and/or NR1H2 receptor protein homologue mediated disease or condition in a mammal comprising administering a therapeutically effective amount of a compound according to any of claims 1 to 12, wherein the prevention or treatment is directly or indirectly accomplished through the binding of the compound according claims 1 to 13 to the NR1H3 and/or NR1H2 receptor proteins or to the NR1H3 and/or NR1H2 receptor protein homologues.

15. A method for prevention or treatment of a NR1H3 receptor protein and/or NR1H2 receptor protein mediated disease or condition according to claim 14, wherein said mammal is a human.

16. A method for regulating the cholesterol synthesis and/or transport in a mammal which comprises activating the NR1H3 and/or NR1H2 receptors with a therapeutically effective amount of a compound according to claims 1 to 12.

17. A method of treating in a mammal a disease which is affected by cholesterol, triglyceride, or bile acid levels comprising administering to a mammal in need of such treatment a therapeutically effective amount of a compound according to claims 1 to 12.

18. A method of treating atherosclerosis, alzheimers disease, lipid disorders, obesity or a cardiovascular disorder in a mammal, in particular a human, comprising administering to a mammal in need of such treatment a therapeutically effective amount of a compound according to claims 1 to 12.

19. A method according to any of claims 14 to 18 wherein the expression of ABCA 1 and/or ABCG1 and/or ABCG5 and/or ABCG8 is increased.

20. A method according to any of claims claim 14 to 19 wherein the expression of the cholesterol 7 α hydroxylase and/or the activity of the cholesteryl ester transfer protein is increased.

21. A method of blocking in a mammal the cholesterol or fatty acid absorption in the intestine of a mammal in need of such blocking comprising administering to a mammal in need of such treatment a therapeutically effective amount of a compound according to claims 1 to 12.

22. A method for treating obesity in a mammal comprising administering a therapeutically effective amount of a compound according to any of claims 1 to 12.

23. A method of modulating a gene whose expression is regulated by the NR1H3 and/or NR1H2 receptor in a mammal comprising administering a therapeutically effective amount of a compound according to claims 1 to 10.

24. A method according to any of claims claim 14 to 19 wherein the expression of the cholesterol 7 α hydroxylase and/or the activity of the cholesteryl ester transfer protein is enhanced.

25. Use of a compound according to any of claims 1 to 12 wherein the mammal is a human

26. Use of a compound according to any of claims 1 to 12 for the manufacture of a medicament for the prevention or treatment of a NR1H3 and/or NR1H2 receptor protein or NR1H3 and/or NR1H2 receptor protein homologue mediated disease or condition in a mammal wherein the prevention or treatment is directly or indirectly accomplished through the binding of the compound according claims 1 to 8 to the NR1H3 and/or NR1H2 receptor protein or NR1H3 and/or NR1H2 receptor protein homologue.

27. Use of a compound according to any of claims 1 to 12 for the manufacture of a medicament for prevention or treatment of a NR1H3 and/or NR1H2 receptor protein mediated disease or condition according to claim 26, wherein the mammal is a human.

28. Use of a compound according to any of claims 1 to 12 for the manufacture of a medicament for regulating the cholesterol transport system in a mammal by activating the NR1H3 and/or NR1H2 receptor.

29. Use of a compound according to any of claims 1 to 12 for the manufacture of a medicament for regulating levels of cholesterol, triglyceride, and/or bile acid.

30. Use of a compound according to any of claims 1 to 12 for the manufacture of a medicament for treating in a mammal atherosclerosis, alzheimer disease, gallstone disease, lipid disorders, obesity or a cardiovascular disorder.

31. Use of a compound according to any of claims 1 to 12 for the manufacture of a medicament capable for blocking in a mammal the cholesterol and/or fatty acid absorption in the intestine.

32. Use of the compound according to any of claims 1 to 12 for the manufacture of a medicament for treating obesity in a mammal.

33. Use of a compound according to any of claims 1 to 12 for the manufacture of a medicament for modulating a gene whose expression is regulated by the NR1H3 and/or NR1H2 receptor.

34. Use of a compound according to any of claims 1 to 12 in a mammal for the selective up-regulation of one or more genes selected from the group consisting of ABCA1, ABCG1, ABCG5 and ABCG8 and a down-regulation of one or more of the genes selected from the group comprising FAS and SREBP-1c, said compound showing a larger difference in regulation of the two groups of genes when compared with the regulatory behavior of T0901317 on both groups of genes.

35. Use of a compound according to claims 28, 30, 31, 32, and 34 wherein the mammal is a human.
